# EUROPÄISCHE PATENTANMELDUNG

(11) **EP 3 895 709 A1**
(43) Veröffentlichungstag der Anmeldung: **20.10.2021**
(21) Anmeldenummer: 20170199.2
(22) Anmeldetag: 17.04.2020
(51) Int. Cl.: A61K 31/685, A61P 29/00, A61P 37/02

(54) **PHOSPHOLIPIDE UND PHOSPHOLIPID-METABOLITEN ZUR BEHANDLUNG VON VIRALEN UND BAKTERIELLEN LUNGENENTZÜNDUNGEN UND SEPSIS**

(71) Anmelder: Andreas Hettich GmbH & Co. KG, 78532 Tuttlingen (DE)
(72) Erfinder: Massing, Ulrich, 79249 Merzhausen (DE)
(74) Vertreter: dompatent von Kreisler Selting Werner - Partnerschaft von Patent- und Rechtsanwälten mbB

(57) **Zusammenfassung**

Die Erfindung betrifft die Verwendung von Acylglycerophospholipiden, insbesondere von Acylglycerophosphatidylcholinen sowie deren Metaboliten wie Lyso-Phosphatidylcholine und alpha-Glycerophosphocholine, zur Behandlung, zur Vorbeugung bzw. zur Unterstützung der Behandlung sowie zur Nachbehandlung von viralen und bakteriellen Lungenentzündungen und Sepsis, einschließlich der Lungenentzündung und Sepsis in Folge von Influenza, Covid-19, ARDS, Krebs und Pneumonitis nach Immuntherapie bei Krebs.

## Beschreibung

Die Erfindung betrifft die Verwendung von Acylglycerophospholipiden, insbesondere von Acylglycerophosphatidylcholinen sowie deren Metaboliten wie Lyso-Phosphatidylcholine und alpha-Glycerophosphocholin, zur Behandlung, zur Vorbeugung bzw. zur Unterstützung der Behandlung sowie zur Nachbehandlung von viralen und bakteriellen Lungenentzündungen und Sepsis, einschließlich der Lungenentzündung und Sepsis in Folge von Influenza, Covid-19, ARDS, Krebs und Pneumonitis nach Immuntherapie bei Krebs.

### Hintergrund der Erfindung

Lungenentzündungen sind schwerwiegende Erkrankungen, die insbesondere bei Personen höheren Alters und mit Vorerkrankungen tödlich verlaufen können. Lungenentzündungen können idiopathisch sein, werden aber oft durch Viren als auch durch Bakterien ausgelöst. Oft folgt einer viralen eine bakterielle Lungenentzündung nach. Die Entzündung im Lungengewebe entsteht u.a. durch die oft sehr starke Immunreaktion gegen virale oder bakterielle Erreger im Lungengewebe. Durch die Entzündungsreaktion im Lungengewebe kann u.a. Wasser in die Aveolen eindringen, was den Gasaustausch erschwert. Zudem reduziert sich auch die Menge an Surfactant-Lipiden in den Aveolen (insbesondere von Phosphatidylcholin) und die Oberflächenspannung steigt dementsprechend deutlich an (bis zu 35 mN/m bei Pneumonien und bei ARDS) was das Offenhalten der Alveolen und in Folge die Atemarbeit zusätzlich erschwert (Günther, A. et al., Am. J. Respir. Crit. Care Med., 153(1): 176-184 (1996)).

Die Reaktion des Immunsystems kann bei einer Pneumonie sehr stark ausfallen, so dass ein sog. Zytokinsturm entsteht. Die sehr starke Entzündungsreaktion verläuft dann nicht mehr kontrolliert und der klinische Verlauf gleicht dem einer Sepsis, d. h. dass die Immunantwort ist nicht mehr primär auf das entzündete Organ und die Ursache der Entzündung, z. B. von Viren, ausgerichtet ist, wodurch verschiedene andere Organe und Gewebe teils schwer geschädigt werden können.

Gegen verschiedene Lungenentzündung-auslösende Viren oder Bakterien (z. B. Pneumokoggen oder Influenza-Viren) gibt es Impfungen, die im Einzelfall Nebenwirkungen haben können, wie z. B. eine Impfung gegen Influenza. Ein starkes Ansprechen des Immunsystems durch die Impfung kann grippeähnliche Symptome zur Folge haben, es zeigen sich Symptome wie beispielsweise Frösteln, Müdigkeit, oder Muskelschmerzen.

Bei einer Sepsis läuft ebenfalls, induziert u.a. durch bakterielle Toxine (insbes. bakterielles Lipopolysaccharid (LPS)), eine nicht mehr kontrollierte Immunreaktion ab, in deren Folge es zu einem Organversagen kommen kann, u.a. auch der Lunge, obwohl die Entzündungsreaktion gar nicht auf dieses Organ fokussiert ist. Eine Sepsis oder ein septischer Schock können auch die Folge von Krebs, Polytrauma oder schweren Entzündungen (z. B. Lungenentzündung nach Covid-19 oder Influenza) sein.

Das "Acute Respiratory Distress Syndrome (ARDS)" oder Akutes Atemnotsyndrom ist eine Reaktion bzw. Entzündung der Lunge als Antwort auf schädigende Faktoren, unabhängig davon, ob die pulmonale Entzündung primär pulmonal oder systemisch ausgelöst wird. Wie bei einer Pneumonie kann es zum Einstrom von Wasser in die Lunge, und zur Veränderung bzw. Reduktion des Lungen-Surfactants kommen, wodurch der Gasaustausch gestört wird (Günther, A. et al., Am. J. Respir. Crit. Care Med., 153(1): 176-184 (1996)). Krebserkrankungen werden immer häufiger mit Hilfe von Immuntherapien behandelt. Dabei wird es dem Immunsystem z. B. durch den Einsatz von Checkpoint-Inhibitoren ermöglicht eine Immunantwort gegen Krebszellen auszulösen. Diese vielversprechende Therapie (z. B. mit Ipilimumab oder Nivolumab bzw. Kombinationen der beiden Inhibitoren) hat leider Limitationen. So kommt es zum einen nur bei einem Teil der Krebs-Patienten zu einer Immunreaktion, also einem Ansprechen der Therapie. Bei Versagen der Immuntherapie liegt möglicherweise eine, eventuell durch die Krebserkrankung verursachte Immunschwäche vor, d. h. das Immunsystem kann nicht wie erwartet aktiviert werden. Zum anderen kann die Immunreaktion aber auch außer Kontrolle geraten, ein wichtiges Beispiel ist das Auftreten von Pneumonitis, Kolitis oder Hepatitis, welche alle lebensbedrohlich sein können (Kähler, KC., Pharmakon, 6:463-468 (2018)). Eine Pneumonitis ist eine entzündliche Veränderung der Lunge, die im Gegensatz zur Lungenentzündung nicht durch Mikroorganismen (Bakterien, Pilze) oder Viren ausgelöst wird, sondern durch pneumoschädliche Einflüsse, wobei ionisierende Strahlung, Medikamente oder z. B. chemische oder physikalische Noxen wichtig sind. Um Organschäden nach Auftreten der o.g. Nebenwirkungen zu vermeiden muss die ausgelöste Immunreaktion wieder gestoppt werden, z. B. mit Corticoiden wie beispielsweise Methylprednisolon.

Phospholipide (nachfolgend kurz "PL") sind eine Hauptkomponente tierischer Zellmembranen. Sie bestehen in der Regel aus einem hydrophilen Kopf, der über eine negativ geladene Phosphatgruppe mit hydrophoben unpolaren Resten verknüpft ist. Die in biologischen Membranen häufigsten PL sind Glycerophospholipide.

Glycerophospholipide (nachfolgend "GPL") sind wie in Formel (I) gezeigt aufgebaut:

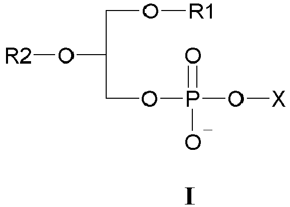

Sie bestehen aus einem hydrophilen Kopf, der über eine negativ geladene Phosphatgruppe in *sn*-3-Position mit einem Glycerinrückgrat und darüber mit einem oder zwei hydrophoben unpolaren Resten R¹ und R² verknüpft ist (IUPAC Compendium of Chemical Technology, 2nd Ed. (1997)). Letztere sind in der Regel *O*-Acylreste aus Fettsäuren mit einer Länge von 14-24 C-Atomen, können jedoch auch *O*-Alkyl- oder *O*-1-Alkenylreste sein. GPL mit einem oder zwei *O*-Acylresten werden auch als 1-Acyl-, 2-Acyl- bzw. 1,2-Diacylglycerophospholipide bezeichnet oder pauschal als Acylglycerophospholipide (nachfolgend kurz "AGPL") zusammengefasst. Typische AGPL sind Phosphatidylglycerin, Phosphatidylserin, Phosphatidylethanolamin (nachfolgend "PE"), Phosphatidylinositol (nachfolgend "PI"), Phosphatidsäure (nachfolgend "PA") und Phosphatidylcholin (nachfolgend "PC"). Acylglycerophospholipide sind insbesondere in Lecithin enthalten, die Hauptkomponente ist hier meist PC.

Glycerophospholipide, insbesondere PC oder PC-enthaltende Mischungen wie z. B. Lecithin, finden wegen ihrer Emulgatorwirkung Einsatz als Bestandteil von Nahrungsergänzungsmitteln und Lebensmitteln, in der Kosmetik, in der parenteralen Ernährung und als Hilfsstoffe zur Formulierung von Arzneimitteln. Meist werden dabei nicht hydrierte Phospholipide aus Soja oder Ei verwendet, lediglich zur Formulierung von Medikamenten für die intravenöse Applikation werden hydrierte Phospholipide bevorzugt.

Glycerophospholipide sind auch als aktive Bestandteile von Nahrungsergänzungsmitteln und von Arzneimitteln zu finden (z. B. Marine Phospholipide (aus Lachsrogen oder Krill)), die einen hohen Anteil langkettige ω-3-Fettsäuren enthalten und z. B. zur Unterstützung der Therapie von Prostatakrebs oder zur Behandlung der Kachexie bekannt sind (EP-A-1745788). Weitere Beispiele sind Soja-Phospholipide, z. B. enthalten in Essentiale® zur Therapie von Lebererkrankungen, in Buer-Lecithin plus Vitamine®, zur Therapie von Erschöpfungszuständen und zur Stärkung der Nerven oder in Lipostabil®, zur Therapie von erhöhten Fettwerten, wobei die die empfohlenen Dosen von 1,5 g bis ca. 6,5 g PL/Tag reichen (Daten jeweils aus den Packungsbeilagen).

Die Resorption von Glycerophosphocholinen im Gastrointestinaltrakt nach ihrer oralen Aufnahme erfolgt nicht direkt. PC wird zunächst von der pankreatischen Phospholipase A2 in LysoPC und freie Fettsäuren gespalten, die beide von der Mucosazelle im Darm (Enterozyt) aufgenommen werden. In der Mucosazelle wird der größere Teil des LysoPCs weiter hydrolysiert, ca. 1/3 wird nach dem aktuellen Stand des Wissens zu PC reacyliert und anschließend Bestandteil von Chylomikronen, die in das Lymphsystem abgegeben werden (Parthasarathy, S. et al., Biochem. J., 140(3): 503-508 (1974)).

Weiterhin ist hydriertes PC bekannt zur Verminderung der Tumormetastasierung (als Liposom zur i. v.-Applikation). Eine Wirkung auf das Immunsystem ist nicht beschrieben.

Desweiteren werden Phospholipide zur Formulierung von Zubereitungen zur parenteralen Ernährung verwendet. Derartige Zubereitungen sind Emulsionen und bestehen typischerweise zu 10, 20 oder 30 % aus Triglyceriden (z. B. Sojaöl, MCT (medium chain triglyceride, mittelkettige Triglyceride), Olivenöl, Fischöl oder deren Mischungen (z. B. Soja/Olive 4:1)). Die Emulsionen enthalten desweiteren einen geringen Anteil Phospholipide (Lecithin), z. B. aus Hühnerei als Emulgatoren, wobei ein möglichst geringes Phospholipid/Triglycerid-Verhältnis angestrebt wird (z. B. Intralipid, Baxter, 10 % (20 %; 30 %) Emulsion: 10 g (20 g; 30 g) Sojaöl und 0,6 g (1,2 g; 1,2 g) Phospholipid/100 ml; Lipundin 10 % N, Braun: 8 g Lecithin auf 100 g Sojaöl; Lipofundin MCT 10%, Braun: 8 g Lecithin auf 100g Soja/MCT (1:1)). Emulsionen mit höherem Triglyceridgehalt benötigen zur Emulgation offensichtlich weniger Phospholipid, was als vorteilhaft angesehen wird. Bei niedrigem Phospholipid/Trigycerid-Verhältnis zeigt sich eine bessere metabolische Verträglichkeit mit signifikant geringerer Akkumulation von Phospholipiden und Cholesterol im Plasma (Hartig, W. et al. (Hrsg.), Ernährungs- und Infusions-therapie, 8. Aufl., Thieme (2004)). Die verwendeten Lecithine sind hoch-angereicherte Lecithine mit einem Glycerophosphatidylcholin-Anteil von typischerweise 75 oder 80 % (z. B. Lipoid E 75/E 80). Alpha-Glycerophosphocholin (alpha-GPC) besteht aus einem Glycerin-Grundkörper an dessen sn-3-Position eine Phosphocholin-Gruppe als Phosphodiester gebunden ist. Formal leitet sich alpha-GPC vom Phosphatidylcholin ab, bei dem die beiden Fettsäurereste durch Hydrolyse entfernt wurden.

Alpha-GPC wird als Wirkstoff in Nahrungsergänzungsmitteln eingesetzt und dient dort als Cholin-Quelle. Alpha-GPC wirkt positiv auf den Zuwachs von Körperkraft durch Training (Bellar, D. et al., J. Int. Soc. Sports Nutr. 12:42 (2015); Marcus, L. et al., J. Int. Soc. Sports Nutr. 14:39 (2017)), und zeigt positive Wirkungen bei Patienten mit Alzheimer und anderen Demenzerscheinungen (Review: Parnetti, L. et al., Mech. Ageing Dev. 122(16) 2041-2055 (2001)) und verbessert die kognitiven Eigenschaften (Suchy, J. et al., Nutr. Res. 29(1) 70-74 (2009)) sowie die Aufmerksamkeit (Hoffman, JR. et al., J. Int. Soc. Sports Nutr. 7:39 (2010)). Es wird diskutiert ob Alpha-GPC den Alterungsprozess verlangsamt (Matsubara, K. et al., Biosci. Biotech. Biochem. 82(4) 647-653 (2017)). Typische Dosen liegen bei 500 - 1.200 mg/Tag. Ein Effekt auf das Immunsystem oder eine Wirkung auf Krebserkrankungen inklusive der Begleiterscheinungen wir Kachexie oder Fatigue sind nicht bekannt.

In Hefen (Saccharomyces cerevisiae) dient alpha-GPC zudem direkt als Vorstufe bei der Biosynthese von LysoPC. Hier wurde eine GPC-Acyltransferase (GPCAT oder Gpc1) beschrieben, die Acyl-CoA-abhängig alpha-GPC acyliert (Stalberg, K. et al., J. Lipid Res. 49: 1794-1806 (2008); Anaokar, S. et al., J. Biol. Chem. 25;294(4):1189-1201 (2018)). Das neue Enzym aus Hefe wurde bereits kloniert und zeigt wenig Verwandschaft mit anderen Acyltransferasen aus dem Lipidbereich (Glab, B. et al., J. Biol. Chem. 291(48): 25066-25076 (2016)). Die Aktivität dieses Enzyms wurde auch in der Färberdistel nachgewiesen. GPCAT-Aktivitäten in tierischem oder humanem Gewebe wurden noch nicht nachgewiesen.

1- bzw. 2-Acyl-glycerophospholipide werden auch als Lyso-Phospholipide (LysoPL) bezeichnet. Lyso-Phospholipide sind einkettige Phospholipide und entstehen aus 1,2-Diacyl-Phospholipiden durch Phospholipase A-katalysierte Abspaltung oder durch chemische Hydrolyse eines Fettsäurerestes. LysoPL haben oberflächenaktive Eigenschaften, welche rote Blutkörperchen zu lysieren vermögen (daher der Name). Aufgrund dessen werden bei Applikation der reinen Lyso-Phospholipide in relevanten Dosen hämolytische Eigenschaften angenommen wie es auch verschiedene, zur Krebsbekämpfung eingesetzte Phospholipide, die nicht Acylglycerophospholipide sind, gezeigt haben (US 4,562,005; US 4,775,758; EP 0171968; US 5,489,580; US 6,172,050; WO 01/72289; DE 4408011; Zeisig, R. et al., Anticancer Drug Des. 16(1):19-26 (2001); Arndt, D. et al., Breast Cancer Res. Treat. 43(3):237-46 (1997); DE-A-3304870; US 4,492,659; DE-A-3204735; US 4,565,659; Modolell, M. et al., Cancer Res. 39(11):4681-4686 (1979)).

Lyso-Phospholipide (1-Acyl- oder 2-Acyl-glycerophospholipide) können aus den verschiedenen zweikettigen, membranbildenden Phospholipiden entstehen. Beispiele für Lyso-Phospholipide sind Lyso-Phosphatidylcholin, Lyso-Phosphatidylethanolamin (Lyso-Kephalin), Lyso-Phosphatidylglycerol, -serin und Lyso-Phosphatidsäure, deren Ausgangs-Phospholipide sämtlich 1,2-Diacylglycero-phospholipide sind. Lyso-Phospholipide sind auch als karzinostatische Verbindungen bekannt (US 4,372,949).

Im Gegensatz zu doppelkettigen Phospholipiden werden Lyso-Phospholipide derzeit nicht als therapeutische Agentien eingesetzt. Es ist jedoch bekannt, dass jede Formulierung mit einem doppelkettigen Phospholipid auch immer geringe Anteile seines entsprechenden natürlichen Abbauproduktes, nämlich dem entsprechenden Lyso-Phsopholipid enthält.

LysoPC ist ein zentrales Stoffwechselprodukt im menschlichen Körper. Der aktuelle Wissensstand zur Entstehung von LysoPC im menschlichen Körper ist, dass LysoPC bei der Abspaltung einer Fettsäure aus dem häufig vorkommenden Phosphatidylcholin (Lecithin) entsteht. PC wiederum wird durch Reacylierung von LysoPC oder via Kennedy-Pathway oder dem Phosphatidylethanolamin-Pathway bereitgestellt (Lands, WEM. et al., J. Biol. Chem. 231(2): 883-888 (1958); Kennedy, EP. et al., J. Biol. Chem., 222(1): 193-214 (1956); Jacobs, RL. et al., J. Biol. Chem. 285(29): 22403-22413 (2010)). Andere Biosynthesewege für LysoPC oder PC im Menschen sind nicht beschrieben.

Aufgrund der Verteilung von gesättigten und ungesättigten Fettsäuren in Phospholipiden (sn1-Position: überwiegend gesättigte Fettsäuren, sn2-Position: überwiegend ungesättigte Fettsäuren) sollte bei Entstehung von LysoPC aus Phospholipiden durch Abspaltung einer (überwiegend ungesättigten) Fettsäure aus der sn-2-Position eines PCs durch Phospholipase A2 überwiegend gesättigtes LysoPC entstehen. Allerdings findet sich überraschenderweise im Plasma nur ein sehr leichter Überschuss an gesättigten LysoPC-Spezies (z. B. Zhao, Z. et al., J. Clin. Oncol. 25(19) 2696 ff (2007: 162 µM gesättigtes LysoPC (55%) vs. 129 µM ungesättiges LysoPC (45 %)).

Wie dem nachfolgenden Diskurs zu entnehmen ist, fokussieren Forschungsarbeiten über LysoPC im Menschen auf die Konzentration dieses Moleküls im Plasma bzw. Serum, wo es überwiegend an Albumin gebunden vorliegt. Albumin verfügt über 3-4 Bindungsstellen für LysoPC und bindet ca. 80 % des Plasma-LysoPCs (Switzer, S et al., J. Lipid Res. 6: 506-511 (1965)).

Der mittlere Wert von LysoPC im Plasma von Menschen ist in verschiedenen Studien untersucht worden - hier einige Beispiele: 280 µM (Drobnik, W. et al., J. Lipid Res. 44: 754-761 (2003); 292 ± 74 µM (Zhao, Z. et al., J. Clin. Oncol. 25(19) 2696 ff (2007); 234 µM (Kishimoto, T., et al., Clin. Biochem. 35: 411-416 (2002). Einige Autoren haben für gesunde Frauen etwas niedrigere Werte als für Männer gefunden (365 vs. 340 µM: Süllentrop, F. et al., NMR Biomed. 15(1) 60-68 (2002); 270 vs. 230 µM: Gillett, MPT. et al., Atherosclerosis 22: 111-124 (1975). Die LysoPC-Werte in den jeweils untersuchten Kollektiven gesunder Probanden hatten immer eine beträchtliche Schwankungsbreite, was auf individuell unterschiedliche "optimale LysoPC-Werte" bei unterschiedlichen Menschen schließen lässt. Nichts desto trotz scheint es vorteilhaft für einen Menschen, wenn er tendenziell eher hohe LysoPC-Werte aufweist. So wurde gefunden, dass hohe Spiegel an LysoPC-Spezies, welche als Fettsäure Stearinsäure beinhalten, zu einem geringeren Risiko für Brustkrebs, Prostatakrebs sowie für colorektale Tumoren führen (Kühn, T. et al., BMC Medicine 14:13 (2016). Zudem wurde gezeigt, dass die Serum-LysoPC-Werte bei langlebigen Menschen hoch signifikant erhöht sind (Montoliu, I. et al., Aging 6(1) 9-25 (2014) - supplementary tables).

Aus den oben genannten Studien ergibt sich ein ungefährer mittlerer LysoPC-Plasma-Wert in Gesunden von ca. 300 µM, was z. B. für einen Mann mit einem Plasmavolumen von ca. 3,5 I bedeutet, dass sich ca. 500 mg LysoPC im Plasma befinden. Die LysoPC-Halbwertszeit im Menschen wurde noch nie bestimmt, liegt aber wahrscheinlich im Bereich weniger Stunden. Im Totenkopfäffchen wurde die Halbwertszeit mit 1,3 h bestimmt (Portman, OW. et al. J. Lipid Res. 11:596-604 (1970)), in der Maus mit ca. 4 h (Dissertation Anna Raynor, Uni-Freiburg, 2015)). Bei einem geschätzten Wert für den Menschen von 2,5 h ergäbe sich ein Umsatz von ca. 2-3 g LysoPC/d.

Die Entstehung von LysoPC im Menschen ist nicht umfassend bekannt. Bekannt ist, dass LysoPC u.a. durch Hydrolyse von Phospholipiden in Lipoproteinen entsteht, z. B. durch die Enzyme LCAT (Lecithin-Cholesterol-Acyl-Transferase) oder durch die endotheliale Lipase. Angenommen die komplette PL-Hülle von Chylomikronen (enthalten max. 10 % PL) wird zu LysoPL hydrolysiert, entspricht dies bei einer Fettaufnahme von ca. 50 g Fett (Triglycerin) ca. 5 g PL, was ca. 3-4 g LysoPL entsprechen würde. Maximal 6-12 g LysoPC könnten aus der Hydrolyse von Phosphatidylcholin aus der Gallenflüssigkeit entstehen, wenn die 10-20 g PC (Northfield, TC., et al. Gut, 16:1-17 (1975)), die täglich mit der Gallenflüssigkeit in den GI-Trakt gelangen, zu LysoPC hydrolysiert würden und diese dann auch vollständig resorbiert würden, was nicht der Fall ist (s.u.). Hinzu kämen noch wenige Milligramm bis zu einigen Gramm LysoPC durch Spaltung von Phospholipiden aus der Nahrung im GI-Trakt. So enthält ein Ei ca. 1 g PC. Wie oben beschrieben wird allerdings ein größerer Anteil des LysoPCs in der Mucosazelle weiter hydrolysiert, nur ca. 1/3 wird wieder PC reacyliert und anschließend Bestandteil von Chylomikronen, die in das Lymphsystem abgegeben werden (Parthasarathy, S. et al., Biochem. J., 140(3): 503-508 (1974)). Die direkte Abgabe von LysoPC ins Blut oder ins Lymphsystem ist nicht beschrieben. Auch der Verbrauch von LysoPC im Körper wurde noch nicht umfassend erforscht, allerdings sind einige Prozesse bekannt oder wahrscheinlich. So wurde bereits gezeigt, dass die Versorgung des ZNS mit langkettigen und hochungesättigten Fettsäuren sowie (wahrscheinlich) mit Cholin durch die spezifische Aufnahme von langkettigen und hochungesättigten LysoPC-Spezies erfolgt (Bernoud, N. et al., J. Neurochem. 72:338-345 (1999)). Kürzlich wurde ein Natrium-abhängiger LysoPC-Transporter des ZNS gefunden und charakterisiert (MFSD2A), der das erste Mitglied einer Superfamilie von LysoPC-Transportern zu sein scheint (Nguyen, LN. et al. Nature 509:503-506 (2014)). Lungensurfactant wird von den Alveolarmakrophagen Typ II hergestellt und besteht zu einem großen Teil aus gesättigtem Phosphatidylcholin sowie den Surfactantproteinen A bis D (Agassandian, M. et al., BBA 1831(3): 612-625 (2013). Lungensurfactant breitet sich über die feuchte Oberfläche der sehr kleinen Alveolen aus und repräsentiert die Grenze zur Luft. Surfactant reduziert dabei drastisch die Oberflächenspannung des alveolaren Wassers und trägt hauptsächlich dazu bei, dass die Alveolen nicht aufgrund einer zu hohen Oberflächenspannung kollabieren und dass die Atemarbeit (zum Aufblähen der Alveolen) gering bleibt.

Lungen-Surfactant besteht zum Großteil aus Phosphatidylcholin (PC) und wird zur Aufrechterhaltung des Gasaustausches in den Aveolen von den Alveolarmakrophagen Typ II ständig erneuert (Goss, V. et al. BBA 1831: 448-458 (2013)). Das hierzu notwendige PC wird dabei durch einfache Reacylierung aus LysoPC hergestellt werden, das zentrale Enzym bei der Biosynthese der gesättigten PC-Spezies ist LPCAT 1 (Lyso-Phosphatidylcholin-Acyltransferase 1), welches LysoPC unter Verbrauch von AcylCoA in ein PC umwandelt (Agassandian, M. et al., BBA 1831(3): 612-625 (2013)). Es liegt sehr nahe, dass sowohl LysoPC aus den Alveolarmakrophagen selbst als auch aus der Peripherie für den Aufbau von Lungen-Surfactant eine Rolle spielen. In Experimenten mit Totenkopfäffchen wurde gefunden, dass bei Gabe von radioaktiv markiertem LysoPC eine große Menge der Aktivität in der Lunge akkumuliert (Portman, OW. et al. J. Lipid Res. 11:596-604 (1970)). Zudem ist vorstellbar, dass bei niedrigen LysoPC-Spiegeln der Schutz der Lunge gegen Infektionen nachlässt, der auch über Aleovarmakrophagen Typ II geregelt wird (d. h. die gleichen Zellen die das Lungen-Surfactant produzieren, prozessieren und präsentieren auch die Pathogene und leiten so die Immunantwort in den Alveolen ein (American Journal of Respiratory & Critical Care Medicine, 2008: 179(5), pp 344-55*)).*

Im Totenkopfäffchen wurde nach Gabe von radioaktiv markierten LysoPC eine sehr hohe Aufnahme von Radioaktivität in den (?) Nieren gefunden (Portman, OW. et al. J. Lipid Res. 11:596-604 (1970)). Wozu LysoPC in der Niere benötigt wird ist unklar.

LysoPC scheint zudem insbesondere bei pathologischen Prozessen in größerer Menge benötigt zu werden, da bei vielen Krankheiten und besonderen Lebensumständen erniedrigte LysoPC-Spiegel beobachtet wurden. Beispiele sind Menschen mit Krebserkrankungen, Patienten mit starken Immunreaktionen (Sepsis), Diabetiker (Typ 2) und Übergewichtige (Barber, MN. et al. Plos one 7(7): e41456 (2012)) oder auch Ältere (Johnson, AA. et al., Aging Cell, 18(6): e13048 (2019)).

In diversen wissenschaftlichen Publikationen wurde gezeigt, dass LysoPC-Werte in Krebspatienten fast durchgehend erniedrig sind, insbesondere bei fortgeschrittenen Krebserkrankungen. So hat z. B. Zhao et al. Werte bei CRC-Patienten von 228 µM gefunden (Kontrollen: 292 µM) (J Clin Oncol 2007, 25(19):2696), bei Taylor et al. wurden bei verschiedenen Tumorentiäten 207 µM gesehen (Lipids Health Dis. 2007, 6:7). Die Varianzen der Werte sind bei Krebs-patienten höher als bei den gesunden Kontrollen. Die LysoPC-Spiegel werden weiter erniedrigt, wenn sich die Patienten nicht ausreichend ernähren können, z. B. durch eine Appetitstörung, Kachexie, etc. (Lipids Health Dis. 2007, 6:7) Mittlerweile hat sich gezeigt, dass ein wichtiger Grund für die erniedrigten LysoPC-Spiegel bei Krebspatienten ein erhöhter LysoPC-Umsatz ist, da insbesondere solide Tumorzellen sehr rasch größere Mengen an LysoPC abbauen, u.a. zur Nutzung als Energieträger (Lipids Health Dis. 2015, 14:69). So ergibt eine Kalkulation auf Basis von Zellkulturdaten einen LysoPC-Abbau von ca. 1 g LysoPC/g aktiver Tumorzellen pro Tag, es kann also bei entsprechend aggressiven und/oder großen Tumoren zu einer dramatischen Abnahme des LysoPC bei gleichzeitiger Erhöhung des Gesamtumsatzes kommen.

In EP 050365 wurde der therapeutische Einfluss von Phospholipiden auf die tumorinduzierte Kachexie beschrieben. Die Tumorkachexie ist ein häufiges Syndrom bei Tumorpatienten mit starker Gewichtsabnahme und Änderung der Körperzusammensetzung. Es kommt zum Abbau des Fettgewebes, der Skelettmuskulatur und zur Beeinträchtigung immunologischer Abwehrmechanismen (Tisdale, M.J., Nutrition 17(5):438-442 (2001); Tisdale, M.J., Curr. Opin. Clin. Nutr. Metab. Care 5(4):401-405 (2002)). Neben der Sepsis ist die Kachexie eine häufige direkte Todesursache bei Tumorpatienten; Zahlenangaben schwanken zwischen 5 und 25% (Klastersky, J., Eur. J. Cancer, 149 (1972)).

Die tumorinduzierte Kachexie wird als entzündlicher Prozess angesehen. Die systemische Entzündungsreaktion wird wiederum als Ursache des beobachteten Verlustes von Appetit (Inui, A., Cancer Res., 4493 (1999)) und Körpergewicht (Fearon, K.C.H., World J. Surgery, 584 (1999)) gesehen, die parallel auch die Tumorprogression fördern kann (Coussens, L.M. und Werb, Z., Nature, 860 (2002)). Dementsprechend wird in EP 050365 die auch bei Kachexie-Patienten gesehene Abnahme der LysoPC-Spiegel insbesondere mit der systemischen Entzündungsreaktion erklärt, was aber aufgrund des zwischenzeitlich entdeckten Mechanismus des LysoPC-Verlustes bei Krebspatienten - nämlich die Spaltung von LysoPC durch eine LysoPC-Lipase außerhalb der Tumorzellen (Lipids Health Dis. 2015, 14:69) - kritisch zu diskutieren ist. In EP 050365 wird der Einsatz von Phospholipiden zur Behandlung der Kachexie beschrieben, die einzusetzenden Dosen rechen von sehr niedrigen bis zu sehr hohen Mengen an Phospholipiden (2-300 mg/kg).

LysoPC spielt auch eine wichtige Rolle bei Immunreaktionen. So wurde gefunden, dass das Auslösen einer starken systemischen Immunantwort (Sepsis) eine dramatische Verminderung des LysoPC-Spiegels zur Folge hat. So wurde bei einer Studie mit 100 Sepsis-Patienten ein mittlerer LysoPC-Wert von nur 95 µM gemessen (gesunde Kontrollen in dieser Studie: 280 µM), wobei das Sterberisiko bei den niedrigsten LysoPC-Werten am höchsten war (J. Lipid Res. 2003, 44:754-761). LysoPC wurde in Kombination mit weiteren Parametern als prognostischer Faktor für das Überleben bei Sepsis vorgeschlagen.

Nicht nur Sepsis scheint zu einem erhöhten Turnover und damit zu niedrigeren LysoPC-Spiegeln zu führen. In einer Studie wurde LysoPC vor und nach der Operation von colorektalen Karzinomen bestimmt. Die LysoPC-Werte sinken durch die OP und die einhergehende Immunreaktion signifikant ab. Patienten mit postoperativen Komplikationen hatten signifikant niedrigere LysoPC-Werte als die Patienten ohne Komplikationen (Surg Today 2018, 48(10):936-943).

Die Expansion von Immunzellen und einige weitere Immunfunktionen scheinen von LysoPC abhängig zu sein, was durch die extrem starke LysoPC-Reduktion bei Sepsis-Patienten gezeigt wurde (siehe oben). Zudem wurde in vitro gefunden, dass LysoPC die Aktivierung des Immunsystem unterstützt und auch die INFy und TNFa Sekretion induziert. Makrophagen werden aktiviert, ebenso B-Zellen (Huang, YH. et al., Clin. Exp. Immunol. 116: 326-331 (1999); Yamamoto, N. et al., J. Immunol. 147: 273-280 (1991). In vitro wurde an NK-Zellen gezeigt, dass LysoPC die Autophosphorylierung von PKCζ induziert, was zu einer Stimulierung der NK-Zell-Aktivität führte (Toxicon 2007, 50(3):400-410). LysoPC scheint hier in einer Doppelfunktion als Signalmolekül sowie als Metabolit/Baustein für die rasche Immunzellaktivierung und -proliferation notwendig zu sein. Eine ausreichende Menge an LysoPC ist folglich für eine vernünftige Immunreaktion und für das Überleben einer Sepsis sehr wichtig, was in verschiedenen tierexperimentellen Arbeiten bestätigt wurde.

So wurde gezeigt, dass LysoPC (s.c.) dosisabhängig das Überleben von Mäusen in einem E.coli-basierten Sepsismodell sehr stark verbessert (von ca. 20 % auf 90 % Überleben bei 20 mg/kg LysoPC). Zudem konnte gezeigt werden, dass eine Mehrfachgabe über 48 h und damit ein in dieser Zeit dauerhaft erhöhter LysoPC-Spiegel in den Mäusen das Langzeitüberleben am besten erhöhte (eine einmalige Injektion erhöhte den LysoPC-Spiegel in den Mäusen nur für ca. 4 h) (Nature Medicine 2004, 10 (2):161-167).

In einem Rattenmodell des septischen Schocks (Gram-positiv und -negativ) wurde gezeigt, dass LysoPC (bis 10 mg/kg, i. v.) die Organschädigungen und Fehlfunktionen deutlich reduzieren kann, höhere Dosen bis 100 mg/kg erweisen sich als untauglich oder gar schädlich. Die schützenden Effekte von LysoPC gingen mit einer Reduktion der IL1-β-Level einher (Brit. J. Pharmacol 2006, 148:769-777)

In zwei weiteren Sepsis-Modellen in Mäusen wurde gezeigt, dass die Injektion (i. p.) von 25 mg/kg LysoPC das Überleben der Tiere von 0 % auf 69 % bzw. 40 % erhöhte (Antimicrob Agents Chemother 2015, 59:3920-3924), in gleicher Weise verbesserte sich die Bakterien-Clearance. Die Ergebnisse wurde von der gleichen Gruppe ein Jahr später bestätigt, und es wurde zudem noch gezeigt, dass die Gabe verschiedener antibakterieller Substanzen synergistisch die Wirkung von LysoPC verstärkten (Antimicrob Agents Chemother 2016, 60:4464-4470).

Zudem zeigt LysoPC bei gleichzeitiger Gabe mit verschiedenen Antigenen in Mäusen eine Wirkung als Adjuvanz. Die Wirkung von LysoPC bei der Produktion der antigen-spezifischen Antikörper war mit der von Alum identisch. LysoPC induziert auch cytotoxische T-Zellen (Vaccine 2006, 24(9):1254-1263) Obwohl LysoPC eine Rolle bei der Aktivierung einer Immunreaktion spielt, werden nach Aktivierung offensichtlich erhöhte Mengen an LysoPC-Spiegel für die Immunreaktion benötigt, was sich in den oben erwähnten, erniedrigten LysoPC-Spiegeln manifestiert. Werden bei einer sehr starken Immunreaktion, wie z. B. aber nicht begrenzt auf Sepsis, kritisch niedrige LysoPC-Spiegel erreicht, wird offensichtlich ein Teufelskreis ausgelöst. Das (über)aktivierte Immunsystem "verbraucht" viel LysoPC, schafft es aber nicht, den Grund für die Entzündung zu neutralisieren (z. B. Pathogen), was zu einem weiterem Absinken des LysoPC-Spiegels führt. Die kritisch erniedrigten LysoPC-Spiegel reichen für die Versorgung der an sich gesunden Organe dann nicht mehr aus, was zu einem Organversagen beitragen kann. Zudem kann vermutet werden, dass LysoPC auch eine regulierende Rolle in Immunreaktionen spielt, weshalb ein stark erniedrigter Spiegel möglicherweise das Überschießen einer Immunreaktion noch beschleunigt. So ist bekannt, dass LysoPC die Differenzierung regulatorischer T-Zellen induziert und die TGF-β-Produktion anregt (Hasegawa, H. et al., Biochem. Biophys. Res. Commun. 415: 526-531 (2011)).

Die gezeigten Zusammenhänge können auch das oft schlechte Ansprechen von Krebspatienten auf Immuntherapien, z. B. mit Checkpoint-Inhibitoren, erklären, aber auch, warum Immuntherapien häufig aufgrund einer Überreaktion des Immunsystems abgebrochen werden müssen. Der bei einer Immuntherapie aus Gründen des erhöhten Verbrauchs durch Tumorzellen erniedrigte LysoPC-Level in Krebspatienten kann einerseits dazu führen, dass die Immunreaktion nicht richtig aktiviert werden kann, oder aber dass eine starke, im Prinzip gewünschte Immunreaktion aufgrund des zusätzlichen Absenkens der LysoPC-Spiegel zu unerwünschten Nebenwirkungen führt, wie z. B. eine Pneumonitis.

LysoPC-Plasma-Spiegel sind auch bei Patienten mit Übergewicht und/oder Diabetes Typ II erniedrigt (Barber, MN. et al. Plos one 7(7): e41456 (2012), was in kritischen Situationen, z. B. bei einer Sepsis, die Kapazität des Immunsystems (s.o.) schneller erschöpfen könnte als bei normalgewichtigen, nicht diabetischen Patienten. Auch bei älteren Menschen findet sich ein Trend zu niedrigeren LysoPC-Plasma-Spiegeln (Johnson, AA. et al., Aging Cell, 18(6): e13048 (2019)). Zusammenfassend kann gezeigt werden, dass viele, insbesondere lebensbedrohliche Erkrankungen mit einem teils starken Abfall der LysoPC-Spiegel im Körper verbunden sind. Beispiele sind Lungenentzündungen, Sepsis, Influenza, Covid-19, ARDS, oder eine Pneumonitis nach Immuntherapie bei Krebs. Diese niedrigen Spiegel könnten zur Folge haben, dass viele, auf LysoPC angewiesene Organe nicht mehr adäquat versorgt werden können, darunter die Lunge, das ZNS, die Niere u. v. m. Die unterversorgten Organe können geschädigt, in ihrer Leistungsfähigkeit beeinträchtigt werden oder gar versagen. Dies könnte insbesondere Patienten mit Vorerkrankungen und Ältere stärker betreffen, da bei diesen Personen tendenziell häufiger niedrigere LysoPC-Plasma-Spiegel vorkommen.

Die der Erfindung zugrundeliegende Aufgabe war somit, ein Medikament zu entwickeln, das in der Lage ist, den durch die jeweilige Erkrankung hervorgerufene LysoPC-Mangel aufzuheben oder zumindest abzuschwächen, und damit zur Behandlung, zur Vorbeugung bzw. zur Unterstützung der Behandlung sowie zur Nachbehandlung von Lungenentzündungen und Sepsis, einschließlich der Lungenentzündung und Sepsis in Folge von Influenza, Covid-19, ARDS, Krebs sowie einer Pneumonitis nach Immuntherapie bei Krebs geeignet ist. Im Fall der Entzündungskrankheiten soll eine Stärkung des Immunsystems bei gleichzeitiger Vermeidung von Organschäden erreicht werden, was aktuell im Falle von Covid-19 die Schaffung einer Herdenimmunität bei gleichzeitiger Vermeidung von Todesfällen möglich machen könnte.

### Kurzbeschreibung der Erfindung

Es wurde gefunden, dass sich wesentlich größere Mengen an LysoPC im Körper befinden als aus den LysoPC-Plasmakonzentrationen zu schließen ist. Fast das gesamte LysoPC im Körper, ca. 95 %, befindet sich in tieferen Kompartimenten, die mit dem Plasma-Kompartiment in einem schnellen Gleichgewicht stehen. Der LysoPC-Umsatz liegt dadurch um mehr als eine Größenordnung höher als bisher angenommen, grob geschätzt liegt er bei mindestens 50 g pro Tag.

Weiterhin wurde gefunden, dass der LysoPC-Spiegel durch die orale Gabe von 1,2-Acyl-Glycerophosphocholinen (PC) effektiv erhöht werden können. Entgegen der Lehrbuchmeinung ist ein praktisch vollständiger Übergang des oral applizierten PCs als LysoPC in das Plasma und die tieferen Kompartimente möglich - zumindest bei Gabe von größeren Mengen an PC wird das im Darm aus PC gebildete LysoPC überwiegend nicht in den Enterozyten abgebaut und tritt direkt ins Blut über.

Weiterhin wurde gefunden, dass der LysoPC-Spiegel durch die orale oder systemische Gabe von alpha-Glycerophosphocholin erhöht werden könne. Es erscheint möglich, dass alpha-GPC in der Leber durch eine noch nicht beschriebene Acylierungsreaktion direkt in LysoPC umgewandelt wird.

Weiterhin wurde gefunden, dass der LysoPC-Spiegel durch die orale oder systemische Gabe von Glucose erhöht werden kann. Wahrscheinlich wird die Glucose in der Leber letztlich zur Fettsäurebiosynthese verwendet, die gebildeten Fettsäuren werden dann als LysoPC ins System abgegeben.

Weiterhin wurde gefunden, dass LysoPC trotz seiner potentiell hämolytischen Effekte direkt als Infusion ins Blut abgegeben werden könnte. Da LysoPC zum größten Teil nicht im Plasma, d. h. nicht Albumin-gebunden vorliegt, sondern sich in tieferen Kompartimenten (Gewebe) befindet und sich zudem das LysoPC-Plasma-Gewebe-Gleichgewicht sehr schnell einstellt, erscheint die direkte Applikation von LysoPC möglich. Wird LysoPC in großen, therapeutisch relevanten Mengen langsam ins Blut infundiert, wird es dort spontan an Albumin gebunden und verliert so seine hämolytischen Eigenschaften. Vor dort geht es aufgrund des Konzentrationsgradienten sehr schnell auch in tiefere Kompartimente über, und das Albumin steht wieder für die Bindung von LysoPC und damit zur Verhinderung seiner hämolytischen Aktivität bereit.

Schließlich wurde gefunden, dass auch Muskelgewebe einen hohen Verbrauch an LysoPC zeigen, was erklärt, warum es bei einem LysoPC-Mangel, z. B. bei einer Influenza, zu körperlicher Schwäche und auch Herzprobleme kommen kann.

Aus diesen Ergebnissen wurde geschlossen, dass die Applikation von Phospholipiden oder von alpha-GPC geeignet ist, den bei viralen und bakteriellen Lungenentzündungen, ARDS, Sepsis, einschließlich der Lungenentzündung und Sepsis in Folge von Influenza, Covid-19, ARDS, Krebs sowie einer Pneumonitis nach Immuntherapie bei Krebs auftretenden LysoPC-Mangel zu verringern bzw. diesem vorzubeugen. Die Erfindung betrifft somit
(1) Phospholipid- und/oder Alpha-Glycerophosphocholin(alpha-GPC)-haltige Zusammensetzungen zur Behandlung von viralen und bakteriellen Lungenentzündungen und Sepsis, einschließlich der Vorbeugung bzw. Unterstützung der Behandlung und der Nachbehandlung (Rekonvaleszenz) von viralen und bakteriellen Lungenentzündungen und Sepsis, was die Behandlung von Lungenentzündung und Sepsis nach Influenza, Covid-19, ARDS, und Pneumonitis nach Immuntherapie bei Krebs einschließt; und
(2) Ein Verfahren zur Behandlung von viralen und bakteriellen Lungenentzündungen und Sepsis, umfassend das Verabreichen einer Phospholipid- und/oder alpha-GPC-haltigen Zusammensetzungen an einen Patienten mit Bedarf einer solchen Behandlung.

Bevorzugt wird die Gabe von hohen Dosen der Zusammensetzung, oral oder systemisch (i. v.), um schnell den LysoPC-Spiegel zu erhöhen und weil im Entzündungsfall ein höherer Umsatz zu erwarten ist.

Die Erfindung wird im Folgenden näher erläutert.

### Detaillierte Beschreibung der Erfindung

Die erfindungsgemäßen Phospholipid-haltigen Zusammensetzungen für die orale Applikation können Soja-, Ei-PC oder Lecithin, marine Phospholipide, z. B. Phospholipide aus Krill oder aus Lachsrogen oder aus Algen enthalten. Die verwendeten Phospholipide können aus diversen Quellen stammen oder synthetisch hergestellt sein, wobei die PL wenig oder keine ω-6-Fettsäuren enthalten sollen, dafür aber bevorzugt ω-3-Fettsäuren, wie z. B. marine Phospholipide oder Krillöl, oder ω-9-Fettsäuren oder gesättigte Fettsäuren. Sie enthalten somit Acylglycerophospholipide (AGPL), insbesondere Acylglycerophosphatidylcholine sowie deren Metaboliten wie Lyso-Phosphatidylcholine (LysoPC) und alpha-Glycerophosphocholin (alpha-GPC) wie nachfolgend näher definiert: Ein "Acylglycerophospholipid" ("AGPL") im Sinne der vorliegenden Erfindung ist z. B. ein 1,2-Diacylglycerophospholipid, 1-Acylglycerophospholipid oder 2-Acylglycerophospholipid mit gesättigten oder ungesättigten Acylresten, einschließlich Phosphatidylcholin, Lyso-Phosphatidylcholin und Lecithin, oder deren pharmazeutisch geeigneten Salzen. AGPL besitzt vorzugsweise die Struktur der Formel (I) 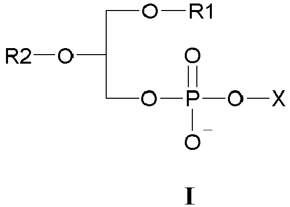 worin
R¹ und R² unabhängig voneinander ausgewählt sind aus H, Alkylcarbonyl-, Alkenylcarbonyl-, Alkinylcarbonyl-, Arylalkylcarbonyl- und Cycloalkylcarbonylresten, worin die Alkylreste geradkettig, verzweigt oder zyklisch, gesättigt oder ungesättigt und mit 1 bis 3 Resten R³ substituiert sein können und in den Alkylresten eines oder mehrere der C-Atome durch O oder NR⁴ ersetzt sein können;
X ausgewählt ist aus H (die Verbindung ist dann eine PA), -(CH₂)ₙ-N(R⁴)₃⁺ (diese Verbindungsklasse umfasst PE und PC), -(CH₂)ₙ-CH(N(R⁴)₃⁺)-COO⁻ (diese Verbindungsklasse umfasst PS) und -(CH₂)ₙ-CH(OH)-CH2OH (diese Verbindungsklasse umfasst PG), wobei n eine ganze Zahl von 1 bis 5 ist;
R³ unabhängig vom Auftreten weiterer R³-Reste ausgewählt ist aus H, Niederalkyl (worin die Niederalkylreste geradkettig, verzweigt oder zyklisch, gesättigt oder ungesättigt sein können), F, Cl, CN und OH; und
R⁴ unabhängig vom Auftreten weiterer R⁴-Reste ausgewählt ist aus H, CH₃ und CH₂CH₃,
oder ein pharmakologisch geeignetes Salz desselben.

Die Acylreste sind bevorzugt Alkylcarbonylreste. Diese können gesättigt oder ungesättigt und gleich oder unterschiedlich lang sein, bevorzugt sind Kettenlängen von C10 bis C24, besonders bevorzugt Kettenlängen von C14 bis C22. Ungesättigte Alkylcarbonylreste sind bevorzugt ausgewählt aus ω-3 und ω-9 Fettsäuren, insbesondere aus Ölsäure (18:1), α-Linolensäure (18:3), Eicosapentaensäure (20:5) und Docosahexaensäure (22:6).

Für den erfindungsgemäßen Einsatz bevorzugt sind AGPL mit natürlich vorkommenden Kopfgruppen, insbesondere Phosphatidylcholine, Phosphatidylethanolamine, Phosphatidylglycerine, Phosphatidylserine und Phosphatidsäuren, ganz besonders APGL ausgewählt aus der Gruppe der Phosphatidylcholine.

In einer besonders bevorzugten Ausführungsform von (1) werden Verbindungen mit der Struktur der Formel (I) oder deren pharmazeutisch geeignete Salze verwendet,
(i) die Alkylcarbonylreste 10 bis 24 C-Atome aufweisen, gesättigt sind oder eine oder mehrere Doppelbindungen enthalten, wobei die Zahl der C-Atome bevorzugt ein Vielfaches von 2 ist und die Doppelbindungen nicht konjugiert sind, und wobei die Alkylreste besonders bevorzugt Fettsäurereste sind;
(ii) die Niederalkylreste 1-3 C-Atome aufweisen und bevorzugt gesättigt sind; und
(iii) n eine ganze Zahl von 1 bis 3 ist.

Ganz besonders bevorzugt sind Verbindungen der Formel (I), worin
(i) R¹ und R² unabhängig voneinander H oder unverzweigte und unsubstituierte Alkylcarbonylreste sind, welche entweder gesättigt sind und dann bevorzugt ausgewählt sind aus Lauryl- (n-Dodecanyl-), Myristyl- (n-Tetradecanyl-), Palmitoyl- (n-Hexadecanyl-), Stearyl- (n-Octadecanyl-), Arachinyl- (n-Eicosanyl-), Behenyl- (n-Docosanyl-) und Lignoceryl- (n-Tetracosanyl-)resten und ganz besonders bevorzugt aus Myristyl-, Palmitoyl-, Stearyl- und Arachniylresten, oder ungesättigt sind und dann bevorzugt ausgewählt sind aus Oleyl- (18:1), α-Linolenyl- (18:3), Eicosapentaenyl- (20:5) und Docosahexaenyl (22:6)-Resten;
(ii) R³ H ist; und
(iii) X -(CH₂-)₂-N(CH₃)₃⁺, -(CH₂-)₂-NH₃⁺, oder -CH₂-CH(NH₃⁺)-COO⁻ ist.

Die Herkunft der AGPL (synthetisch oder aus natürlichen Quellen isoliert) ist für ihren erfindungsgemäßen Einsatz irrelevant. Auch hydrierte AGPL können verwendet werden. Erfindungsgemäße AGPL sind auch Lyso-Acylglycerophospholipide, die sich von AGPL mit zwei Acylresten durch das Fehlen eines Acylrestes in der *sn*-1- oder *sn*-2-Position unterscheiden. Eingesetzt werden können auch handelsübliche Gemische von Glycerophospholipiden oder Fraktionen solcher Gemische. Ein Beispiel ist das sog. Lecithin, das mindestens 20 % Phosphatidylcholin enthalten muss. Besonders bevorzugt sind AGPL der Formel (II) 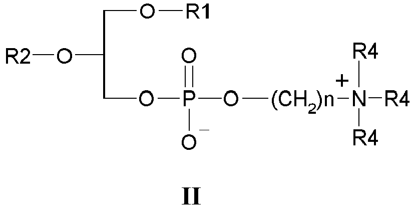 worin
(i) R¹ und R² unabhängig voneinander H oder unverzweigte und unsubstituierte Alkylcarbonylreste sind, welche entweder gesättigt und dann bevorzugt ausgewählt sind aus Lauryl-, Myristyl-, Palmitoyl-, Stearyl-, Arachinyl-, Behenyl- und Lignocerylresten und ganz besonders bevorzugt aus Myristyl-, Palmitoyl-, Stearyl- und Arachniylresten, oder ungesättigt und dann bevorzugt ausgewählt sind aus Oleyl-, α-Linolenyl-, Eicosapentaenyl- und Docosahexaenyl-Resten;
(ii) R⁴ CH₃ oder H ist; und
(iii) n 2 oder 3 ist.

Ganz besonders bevorzugt ist Phosphatidylcholin (PC) mit gesättigten Fettsäureresten, insbesondere Dipalmitoylphosphatidylcholin (DPPC). DPPC ist unter anderem in Lecithin, vor allem in hydriertem Lecithin enthalten. Unter anderem daher ist auch Lecithin sowie hydriertes Lecithin für den erfindungsgemäßen Einsatz geeignet. Ein besonders geeignetes Lecithin ist Ei- oder Soja-Lecithin bzw. hydriertes Ei- oder Soja-Lecithin.

Ist das AGPL ein Lyso-PL, so ist die Fettsäure bevorzugt
(i) eine Fettsäure mit einer Länge von mindestens C16,
(ii) eine gesättigte Fettsäure, oder
(iii) eine ω-3 oder ω-9 Fettsäure, die bevorzugt mindestens eine Länge von C18, besonders bevorzugt von C20 hat und insbesondere Eicosapentaensäure (20:5) oder Docosahexaensäure (22:6) ist.

Ist das AGPL ein Alpha-Glycerophosphocholin (alpha-GPC), so ist es eine der Verbindung der vorstehenden Formel (II), in der beide Reste R¹ und R² Wasserstoff sind, n 2 ist und R⁴ CH₃ ist. Alpha-GPC leitet sich von AGPC ab, wobei aber die die Positionen 1 und 2 nicht substituiert sind. Alpha-GPC ist folglich wasserlöslich und kein Lipid mehr.

"Wirkstoffe" im Sinne der vorliegenden Erfindung sind Verbindungen, die in Lebewesen, insbesondere im Mensch oder Tier, eine physiologische Reaktion hervorrufen können. Sie sind insbesondere in der Therapie eingesetzte Wirkstoffe. Unter einem "pharmakologisch aktiven Wirkstoff" versteht man eine Verbindung, die als Bestandteil eines Arzneimittels die Ursache für dessen Wirksamkeit ist.

Ein "Triglycerid" ist ein Triester des Glycerins mit gleichen oder unterschiedlichen, gesättigten oder ungesättigten Acylresten mit 10 bis 30 C-Atomen. Bevorzugte Triglyceride zur erfindungsgemäßen Verwendung in Kombination mit den AGPL sind gesättigte Fettsäuren und diejenigen ungesättigten Fettsäuren, welche in vivo nicht zu Eicosanoiden, insbesondere nicht zu den biologisch hochwirksamen Eicosanoiden der 2er-Serie wie z. B. PGE₂ umgesetzt werden können. Beispiele hierfür sind gesättigte/hydrierte Triglyceride, MCT, Fischöle bzw. Öle aus der Mikroalge Ulkenia (Ärztezeitung vom 26.08.2004), welche beide sehr viel EPA und DHA enthalten, Olivenöle, Rapsöle, Nachtkerzenöle oder Lein(samen)öle.

In einer bevorzugten Ausführungsform von (1) ist ein 1,2-Diacyl-AGPL der einzige in der Zusammensetzung vorhandene Wirkstoff, besonders bevorzugt das einzige vorhandene GPL. Im letzteren Fall wiederum bevorzugt ist, dass nur 1,2-Acylgylcerophosphocholin vorhanden ist.

In einer anderen bevorzugten Form ist LysoPC der einzige im Medikament enthaltene Wirkstoff.

In einer anderen bevorzugten Form ist alpha-GPC der einzige im Medikament enthaltene Wirkstoff.

AGPL, LysoPC und alpha-GPC können jeweils als einziger Wirkstoff im Medikament vorhanden sein, oder aber als Kombinationen, wobei für die orale Applikation 1,2-AGPL und alpha-GPC und für die systemische Applikation (z. B. i. v.) LysoPC und alpha-GPC bevorzugt sind.

Die Einzelwirkstoffe oder die Kombinationen können als einzige Wirkstoffe im Medikament vorhanden sein, sie können jedoch auch mit weiteren Wirkstoffen kombiniert werden, wobei diese weiteren Wirkstoffe in derselben Zusammensetzung oder als separate Darreichungsform vorliegen können. Möglich sind Kombination der Phospholipidformulierungen oder von alpha-GPC mit Triglyceriden, Fettsäuremonoestern, Wachsen oder freien Fettsäuren, wobei diese Lipide bevorzugt antientzündliche oder zumindest nicht entzündliche Fettsäuren enthalten sollten, d. h. es werden Triglyceride, Fettsäuremonoester, Wachse oder freie Fettsäuren bevorzugt, die wenig oder keine ω-6-Fettsäuren enthalten, aber dafür ω-3- oder ω-9-Fettsäuren oder gesättigte Fettsäuren.

Alpha-GPC (oral oder i. v.) können in Kombination mit den PL (siehe oben) oder mit Fischölen, oder anderen Ölen mit geeigneten Fettsäuren eingesetzt werden. Alle der oben genannten Wirkstoffe in Kombination mit: Albumin (bei system. Gabe); mit Glucose; ggf. mit Insulin; antientzündl. Wirkstoffen; Proteinen/AS, um den Muskelabbau zu reduzieren (bei Krankheit), z. B. Molkeprotein; und Carnithin.

Wichtig im Zusammenhang mit der vorliegenden Erfindung ist, dass die ins System eingebrachten Phospholipide letztendlich verstoffwechselt werden und dass dabei auch an die Phospholipide gebundene Fettsäuren freiwerden. Da ω-6-Fettsäuren wie die Arachidonsäure zu proinflammatorischen Eicosanoiden umgewandelt werden können, werden diese in einer bevorzugten Ausführungsform der AGPL vermieden. AA und seine Vorläuferfettsäure Linolsäure können in das Eicosanoid PGE₂ umgewandelt werden. PGE₂ wirkt nicht nur proinflammatorisch, sondern wirkt autokrin auf Tumorzellen und stimuliert deren Wachstum, ein erhöhter PGE₂-Spiegel ist zudem mit einer erhöhten Metastasierungsrate assoziiert (Attiga, F. A. et al., Cancer Res. 60(16):4629-4637 (2000)). Eine weitere wichtige Implikation eines erhöhten PGE₂-Spiegels ist zudem die Schmerzsensibilisierung, da PGE₂ die Erregbarkeit von Nozizeptoren steigert (Brune, K. und Zeilhofer, H.U., Biospektrum 1:36-38 (2004)).

Wie beschrieben kann ein signifikant erniedrigter LysoPC Level im Körper, verursacht durch eine schwere Entzündungsreaktion oder durch Krebs, möglicherweise Organe schädigen und/oder ihre Funktion beeinträchtigen oder zu Organversagen führen. Daher basiert die erfindungsgemäße Wirkung der genannten Wirkstoffe auf der effektiven Substitution sowie auf der Unterstützung der Neusynthese von LysoPC. Da wir festgestellt haben, dass sehr viel mehr LysoPC als bisher gedacht im Körper vorhanden ist und die Körper-Kompartimente in einem schnellen Gleichgewicht stehen und zudem LysoPC durch weitere wichtige Verbraucher vermindert wird, die nicht nur Lunge und das ZNS umfassen, sondern auch Muskeln und Herz, mussten besonders effiziente Wege gefunden werden dies zu tun. Somit ist die erfindungsgemäße Wirkung der genannten Wirkstoffe an die Gabe ausreichender Mengen der Wirkstoffe gekoppelt, um zumindest eine für die Funktion der Gewebe und Organe ausreichende LysoPC-Menge im Körper zu gewährleisten.

Die erfindungsgemäße Verwendung von Acylglycerophosphocholinen sowie deren Metaboliten LysoPC und alpha-GPC als Wirkstoffe in einem Medikament haben sich als eine überraschend wirksame Methode zur Therapie und zur Unterstützung der Therapie von Lungenentzündungen, Sepsis, Influenza, Covid-19, ARDS, oder einer Pneumonitis nach Immuntherapie und/oder der mit diesen Erkrankungen verbundenen Begleiterscheinungen herausgestellt, dies schließt die Vorbeugung sowie die Unterstützung der Rekonvaleszenz mit ein. So zeigt die vorliegende Erfindung insbesondere, dass die Gabe von AGPL sowie deren Metaboliten die Therapie von Covid-19 unterstützen kann.

Für den hier beschriebenen Therapieansatz zur Behandlung von Lungenentzündungen unterschiedlicher Genese sowie von Sepsis ist Bestimmung der Gesamtmenge von LysoPC im Körper essentiell. Diese ließ sich über die Bestimmung der LysoPC-Konzentration im Plasma - welches mit den tieferen Kompartimenten im Gleichgewicht steht - bestimmen. Hierzu wurde bei einer Testperson zum einen eine hohe Menge Phospholipid aus Ei verabreicht (Beispiel 1 (Bestimmung von Plasma-LysoPC) und Beispiel 2). Das Plasma-Volumen wurde berechnet, der Anstieg der LysoPC-Konzentration bestimmt und die Plasma-LysoPC-Menge, die mit dieser Konzentrationsveränderung einhergeht, berechnet. Diese LysoPC-Menge im Verhältnis zur aufgenommenen Menge ergibt die Verteilung des aufgenommenen LysoPC-Äquivalents zwischen dem Plasma und den tieferen Kompartimenten, woraus sich dann die Gesamt-LysoPC-Menge im Körper berechnen lässt. Es wurde von einer 100 %igen Aufnahme des Phospholipids als LysoPC ausgegangen.

In einem anderen Experiment wurde einer Testperson eine hohe Menge (25 g) reines Triglycerid verabreicht (Beispiel 3). Das Plasma-Volumen wurde berechnet. Bestimmt wurde das Absinken der LysoPC-Konzentration aufgrund des Verbrauchs von LysoPC zur Herstellung von PC für die Biosynthese von Chylomikronen (Ein Chylomikron enthält ca. 5-10 % PC im Verhältnis zur Triglycerid-Menge). Die Plasma-LysoPC-Menge, die mit dieser Konzentrationsveränderung einhergeht, wurde berechnet. Diese Menge an LysoPC im Verhältnis zur aufgenommenen Menge ergibt die Verteilung des aufgenommenen LysoPC-Äquivalenten zwischen dem Plasma und den tieferen Kompartimenten, woraus sich dann die Gesamt-LysoPC-Menge im Körper berechnen lässt. Es wurde von einer 100 %igen Umwandlung von Plasma-LysoPC zu PC ausgegangen.

Durch die Ergebnisse der Beispiele 2 und 3 konnte die LysoPC-Menge in tieferen Kompartimenten mit ca. 95 % des Gesamt-Körper-LysoPCs abgeschätzt werden, d. h. ca. 5 % befinden sich im Blutplasma.

Da der Proband über ca. 3,5 I Plasma verfügte, befindet sich dort bei einer Konzentration von 300 µM (mittlerer Wert für Gesunde, siehe vorn) eine Gesamtmenge von ca. 500 mg LysoPC. Basierend darauf lässt sich die Menge des Gesamt-Körper-LysoPCs mit min 10 g abschätzen.

Basierend auf einer geschätzten Gesamt-Körper-Halbwertszeit für LysoPC von 2,5 h (siehe vorn) ergibt sich ein Tagesumsatz von min 50 g. Allein dieser Wert macht LysoPC zu einem der wichtigen Energieträger im Körper, im Vergleich dazu liegt der Glucoseumsatz bei ca. 200 g/Tag.

Nun wurde der Bedarf an LysoPC bei einem Gesunden durch die aktuell bekannten LysoPC-Verbraucher - die Lunge (Surfactant-Produktion), das ZNS - als nicht ausreichend für einen so hohen Tages-LysoPC-Umsatz von min 50 g eingeschätzt. Daher wurde der LysoPC-Bedarf der anteilsmäßig größten Gewebe-Gruppe, der Muskelmasse, untersucht.

Hierzu (Beispiel 4) wurden bei einer Gruppe von Marathonläufern die Plasma-LysoPC-Spiegel direkt vor und nach dem Lauf untersucht. Bei allen sieben untersuchten Läufern (m/w) fielen die Spiegel sehr deutlich ab, in der Spitze um 45 %, im Mittel um 28 %, was auf einen hohen LysoPC-Verbrauch durch die Muskelmasse hindeutet. Ggf. wirkt LysoPC hier als Fettsäuretransporter, der in der Peripherie der Muskelzellen wieder zu freien Fettsäuren gespalten wird, wodurch ein hoher Fettsäure-Gradient aufgebaut wird, der die Voraussetzung für eine rasche Aufnahme der Fettsäuren via Carnitin ist. Bei der Freisetzung der Fettsäure im Muskelgewebe wird alpha-GPC frei, das als wasserlösliches Molekül wieder in die systemische Zirkulation eintreten kann. Der Transport von Fettsäuren mit Hilfe von LysoPC als Transportform zu Zellen wurde bereits in einem anderen Fall beschrieben, und zwar bei der Fettsäureversorgung von Tumorzellen. Dies ist bei Tumorzellen ein sehr stark ausgeprägter Versorgungsmechanismus und trägt u. a. zu den niedrigen LysoPC-Spiegeln bei Krebspatienten bei (siehe hierzu auch: Hintergrund der Erfindung).

Zudem ist bekannt, dass das Herz ca. 70 % seiner Energie in Form von Fettsäuren aufnimmt. Ob diese Fettsäuren auch in Form von LysoPC zum Herzen transportiert werden ist unklar, aber naheliegend. Daher ist anzunehmen, dass bei Patienten mit entzündungsbedingt stark erniedrigten LysoPC-Spiegeln eine Schädigung des Herzens möglich ist, da letztlich zu wenig Energie zugeführt wird. Dies wird umso wahrscheinlicher als das beobachtet wird, dass viele der aktuell schwer an Covid-19 erkrankten Menschen eine Herzschädigung erleiden, und dass die Vorerkrankung Diabetes das Risiko hierfür steigert (Mohammad Madjid et al., JAMA Cardiology, doi: 10.1001/jamacardio.2020.1286; Riccardo Inciardi et al., doi: 10.1001/jamacardio.2020.1096; Shaobo Shi et al., doi:10.1001/jamacardio.2020.0950, Bonow et al., JAMA Cardiology, doi: 10.1001/jamacardio.2020.1105). Bei Diabetes ist die Aufnahme von Glucose in den Herzmuskel möglicherweise beeinträchtigt. Wird in einem solchen Fall auch noch die Energieversorgung durch Fettsäuren bzw. einem Mangel als LysoPC beeinträchtigt, kann dies möglicherweise zur Schädigung des Herzmuskels beitragen. Eine mögliche Therapieoption wäre hier nicht nur die Erhöhung der LysoPC-Werte, sondern auch die Gabe von Glucose und optional auch von Insulin, um die Glucoseaufnahme zu verbessern.

Obwohl aufgrund der hier gemachten Erkenntnisse ein grundsätzlich hoher LysoPC-Tagesverbrauch sehr plausibel ist, ist nach den Erläuterungen im Kapitel "Hintergrund der Erfindung" der Körper nach Stand es aktuellen Wissens nicht in der Lage, solch hohe Tages-LysoPC-Mengen von mindestens 50 g bereitzustellen. Daher wurde geprüft, ob das bei der Abspaltung von Fettsäuren aus LysoPC freigesetzte alpha-GPC wieder reayliert werden kann, möglicherweise in der Leber als Ort der Fettsäurebiosynthese und als bekannte Quelle für LysoPC, zumindest wenn Albumin zugegen ist, um das LysoPC aus den Hepatozyten außerhalb der Zelle zu binden (Baisted, DJ. et al. Biochem. J. 253: 693-701 (1988)). Eine solche Reacylierungsreaktion von alpha-GPC ist für Menschen und Tiere nicht beschrieben, wohl aber für Hefen (Saccharomyces cerevisiae) (Stalberg, K. et al., J. Lipid Res. 49: 1794-1806 (2008); Anaokar, S. et al., J. Biol. Chem. 25;294(4):1189-1201 (2018))

Beispiel 5 zeigt, dass die orale Aufnahme von 600 mg alpha-GPC bei einem Probanden zur Steigerung der LysoPC-Menge im Körper führt, was die oben genannte Reaktion nahelegt und einen Weg aufzeigt, wie im Körper die notwendigen hohen Mengen an LysoPC bereitgestellt werden können.

In einem weiteren Experiment (Beispiel 6) wurde versucht, die LysoPC-Menge durch Erhöhung der Fettsäurebiosynthese in der Leber zu steigern. Hierzu wurde durch einen Probanden 50 g Glucose aufgenommen, was ebenfalls zu einem raschen Anstieg des LysoPC-Plasma-Spiegels führte.

Somit ist die erfindungsgemäße Wirkung der AGPL und des alpha-GPC bei der Behandlung, Vorbeugung, Unterstützung der Behandlung sowie der Nachbehandlung (Rekonvaleszenz) von Lungenentzündungen, Sepsis, Influenza, Covid-19, ARDS, Krebs oder einer Pneumonitis nach Immuntherapie bei Krebs vermutlich darin begründet, dass die Substanzen den mit diesen Erkrankungen starken Verlust an LysoPC zumindest zum Teil ausgleichen und damit die Immunabwehr verstärken können, auf der anderen Seite ein Überschießen der Immunreaktion verhindern, sowie die Versorgung wichtiger Organe und Muskelgewebe mit LysoPC sicherstellen, insbesondere die Lunge und das Herz. Die erfindungsgemäße Wirkung des AGPL und des alpha-GPC beruht desweiteren vermutlich darauf, dass die zuführten oder im Körper gebildeten LysoPCs *in vivo* letztlich in großen Anteilen wieder in Fettsäuren und alpha-GPC gespalten werden. Diese beiden Komponenten entfalten dann zusätzlich einzeln oder gemeinsam eine Wirkung, welche allein durch die Verabreichung einer der beiden Komponenten nicht problemlos erzielt werden könnte. So kann alpha-GPC wiederum zu LysoPC reacyliert werden und sorgt so - über die initiale Substitution hinaus - für eine längerfristige Stabilisierung des LysoPC-Haushalts. Eine ganz besonders bevorzugte Ausführungsform ist aufgrund des oben gesagten die Verwendung von AGPL (PC oder LysoPC), die als Fettsäuren bevorzugt überwiegend oder ausschließlich hydrierte, ω-3 oder ω-9 Fettsäuren enthalten, und sie enthalten nicht oder nur in geringen Anteilen ω-6 Fettsäuren. Mit anderen Worten: sie enthalten überwiegend oder ausschließlich Fettsäurereste, die *in vivo* nicht zu den biologisch hochaktiven, proinflammatorischen Eicosanoiden, z. B. Prostaglandien E2, umgesetzt werden können. Diese AGPL können u.a. ω-3-haltige Phospholipide sein, wie sie z. B. aus Lachsrogen oder Krill gewonnen werden. Wichtig sind hier auch hydrierte Phospholipide oder LysoPCs, die durch Hydrierung von Phospholipiden aus natürlichen Ressourcen, z. B. aus Ei oder aus Soja gewonnen werden. Möglich sind auch synthetisch hergestellte Phosphatidylcholine wie DPPC.

Eine ganz besonders bevorzugte Ausführungsform ist die erfindungsgemäße Verwendung der AGPL sowie des alpha-GPC in Kombination mit einem Triglycerid oder den aus einem Triglycerid herstellbaren freien Fettsäuren, Mono- und Diglyceriden bzw. von freien Fettsäuren, Wachsen oder Fettsäuremonoestern. Hiervon ist die Verwendung des Triglycerids als weiterer Bestandteil des Medikaments bevorzugt. Besagte Verbindungen sind oder enthalten bevorzugt überwiegend oder ausschließlich hydrierte, ω-3 oder ω-9 Fettsäuren, und sie enthalten nicht oder nur in geringen Anteilen ω-6 Fettsäuren. Mit anderen Worten: sie enthalten oder sind überwiegend oder ausschließlich Fettsäurereste, die *in vivo* nicht zu den biologisch hochaktiven, proinflammatorischen Eicosanoiden, z. B. Prostaglandien E2, umgesetzt werden können. Solche Triglyceride bzw. die resultierenden Di- und Monoglyceride und Fettsäuren sind z. B. gesättigte/hydrierte Triglyceride, MCT, Fischöle bzw. Öle aus der Mikroalge Ulkenia (Ärztezeitung vom 26.08.2004), welche beide sehr viel EPA und DHA enthalten, Olivenöle, Rapsöle, Nachtkerzenöle oder Lein(samen)öle.

Dadurch kann die Wirkung des Medikaments, insbesondere die Wirkung auf die zu behandelnde Entzündung verstärkt werden. Ursache dieses Effekts ist einerseits die bekannte Wirkung von Triglyceriden als hochenergetisches Lebensmittel und Energielieferant. Andererseits ergibt sich aber aus der gleichzeitigen Anwesenheit der Triglyceride und der AGPL bzw. des alpha-GPC ein zusätzlicher Effekt dadurch, dass die zusätzlich z. B. als Triglycerid applizierten gesättigten, ω-3 oder ω-9 Fettsäuren Bestandteil der letztlich gebildeten LysoPCs werden. Dies kann dadurch geschehen, dass im GI-Trakt die Fettsäuren der Triglyceride freigesetzt werden und auch die AGPL in den Enterozyten zumindest zum Teil umgebaut werden, weshalb dann Fettsäuren aus Ölen in die neu synthetisierten AGPL eingebaut werden können. So ist vorstellbar das anstelle der ω-3-haltigen marinen Phospholipide, deren Ressourcen limitiert sind und die teuer sind, ein Gemisch aus AGPL aus z. B. Soja oder Ei, in Kombination mit einem ω-3-haltigen Fischöl gegeben wird, dessen Ressourcen nicht so stark limitiert sind. Weiterhin kann die Gabe entsprechender Öle eine verstärkte Anwesenheit von gesättigten, sowie von ω-3- und ω-9-Fettsäuren in der Leber bedingen, wodurch aus alpha-GPC dann leicht ein LysoPC mit eben diesen Fettsäuren entstehen kann.

Weiterhin werden die besagten gesättigten, ω-3- oder ω-9-Fettsäuren natürlich auch über den ganz normalen Lipidstoffwechsel im Körper verteilt und können so in verschiedenen Organen oder Geweben ihre potentiell antientzündliche Wirksamkeit entfalten und somit synergistisch mit den hier definierten Arzneimitteln wirken.

Wesentlich bei der erfindungsgemäßen Verwendung des AGPL sowie alpha-GPC in Kombination mit einem oder mehreren Triglyceriden ist, dass der AGPL-Anteil am Gesamt-Lipid-Gehalt des Medikaments hoch ist. So sollte dieser AGPL-Anteil mindestens 10 Gew-% betragen, vorzugsweise jedoch mindestens 20 Gew.-%, ganz besonders bevorzugt mindestens 40 Gew-% des Gesamtlipidgewichts. Dies ist gleichbedeutend mit einem Verhältnis AGPL:Triglycerid von 1 : 9, bevorzugt von 2 : 8, besonders bevorzugt von 4 : 6. Grund hierfür ist, dass die AGPL als Wirkstoffe durch die Anwesenheit der Triglyceride in ihrer Wirkung lediglich unterstützt werden. Dies unterscheidet die Medikamente der vorliegenden Erfindung auch von Lipidpräparationen zur künstlichen Ernährung, in denen der PL-Anteil so gering wie möglich gehalten werden muss, sowie von Ölen mit hohen Gehalten an ω-3 oder ω-6-Fettsäuren, bei deren Einnahme es insbesondere um die Erhöhung der genannten Fettsäuren im Körper geht. Des Weiteren können die AGPL in Kombinationen mit Triglyceriden ein weites Spektrum von Fettsäuren (hydriert, ungesättigt) enthalten und aus unterschiedlichsten Quellen stammen (Ei, Soja, Fisch etc.), sie sind also nicht wie die Zubereitungen zur künstlichen Ernährung auf Ei-Lecithin beschränkt.

Wird alpha-GPC als weitere Ausführungsform nicht oral, sondern systemisch, insbesondere i. v. gegeben, dann erfolgt dies bevorzugt als Dauerinfusion, damit keine hohen Peak-Konzentrationen im Blut entstehen, was zur Hämolyse führen könnte. Um dieses Problem weiter zu verringern ist es bevorzugt, alpha-GPC auch in Kombination mit Albumin zu geben, zumindest in den ersten Infusionen, um sicherzustellen, dass der Albumin-Spiegel in den Patienten nicht erniedrigt ist, was wiederum die Gefahr der Hämolyse begünstigt. Zudem ist bekannt, dass die Anwesenheit von Albumin für Freisetzung von LysoPC aus der Leber notwendig ist.

Weiterhin ist es möglich, den Patienten zusätzlich noch Glucose zu geben (i. v. oder oral), damit die Fettsäurebiosynthese in der Leber angeregt wird, was in Kombination mit der Gabe von alpha-GPC oder AGPL (die letztlich in alpha-GPC umgewandelt werden) zu einer verstärkten intrinsischen LysoPC-Synthese führen kann.

Damit die möglicherweise in der Peripherie der Zellen freigesetzten Fettsäuren auch einfach in die Zellen eindringen können, ist eine weitere Ausführungsform der Erfindung die Kombination der Gabe von AGPL und/oder alpha-GPC mit Carnitin, was zur Verbesserung des Fettsäuretranssport in den Zellen beitragen könnte.

Neben den Effekten zu Erhöhung des LysoPC-Spiegels durch die hier genannten Maßnahmen und der damit einhergehenden verbesserten Energieversorgung des Herzgewebes und anderer Organe ist es möglich, insbesondere bei Diabetikern, auch noch Insulin zu geben, damit zusätzlich die optimierte zelluläre Bereitstellung von Glucose die Effekte des LysoPC verstärken kann.

Eine weitere ganz besonders bevorzugte Ausführungsform ist die erfindungsgemäße Verwendung des AGPL in Kombination mit Substanzen, die eine Wirkung auf die Eicosanoidsynthese haben und so die Wirkung des AGPL synergistisch unterstützen können. Erstens sind dies PLA2-Inhibitoren, insbesondere Inhibitoren der sPLA2 (TypII-sPLA, wie sie z. B. in Uhl et al., Phospholipase A2 Basic and Clinical Aspects in Inflammatory Diseases Vol. 24, Karger, Basel, S. 123-175 (1997) und in DE-A-4234130 beschrieben sind).

Zweitens sind dies Substanzen, die die Cyclooxygenasen 1, 2 oder 3 inhibieren, wie die unspezifischen Verbindungen Acetylsalicylsäure, Paracetamol, Diclofenac, Ibuprofen, Metamizol, Phenazon, Propyphenazon sowie COX-2-Inhibitoren, z. B. Meloxicam (Mobec®), Celecoxib (Celebrex®), Rofecoxib (Vioxx®), Etoricoxib (Arcoxia®), Valdecoxib (Rayzon®), und Parecoxib (Dynastat®).

Weiterhin sind dies Substanzen, die im Falle von viralen Infektionen die Ausbreitung des Virus verhindern können, also Virostatika.

Diese Substanzen werden in Konzentrationen/Dosen eingesetzt, wie sie für die therapeutische Verwendung der Substanzen üblich bzw. vom Hersteller vorgeschrieben sind.

Die erfindungsgemäßen Zusammensetzungen sind zur systemischen Gabe geeignet, besonders zur oralen (p. o.) oder intravenösen (i. v.) Gabe.

Die erfindungsgemäße Zusammensetzung kann als Emulsion, als Tabletten, Kapseln oder als Pulver, z. B. zum Einrühren in Nahrungsmittel vorliegen. In der bevorzugten Formulierung als Tablette, Kapsel oder als Pulver kann die Konzentration der AGPL bis zu 100 % betragen.

Die erfindungsgemäße Zusammensetzung kann neben den Phospholipiden, alpha-GPC und weiteren Wirkstoffen noch übliche, pharmazeutisch annehmbare Trägermittel, Hilfsstoffe, Stabilisatoren, Verdünnungsmittel, Bindemittel usw. enthalten.

Die Dosierung der Zusammensetzung wird von dem behandelnden Arzt individuell auf den jeweiligen Patienten angepasst. Sie richtet sich u. a. nach der Art der Krankheit, der Schwere der zu behandelnden Symptome, der konstitutionellen Beschaffenheit des Patienten usw., wobei üblicherweise Dosen von 2-300 mg AGPL/kg Körpergewicht pro Tag und mindestens 500 mg alpha-GPC in Betracht kommen, wobei eine Dosis von mindestens 20 mg AGPL/kg Körpergewicht pro Tag bevorzugt ist. In einer besonders bevorzugten Ausführungsform wird für die Festlegung der optimalen Dosierung die Bestimmung des LysoPC-Spiegels im betroffenen Patienten vorgenommen.

Ein besonderer Vorteil der erfindungsgemäßen Zusammensetzung liegt in ihrer Bedeutung für die Herstellung von Medikamenten zur Behandlung, zur Vorbeugung bzw. zur Unterstützung der Behandlung sowie zur Nachbehandlung (Rekonvaleszenz) von Lungenentzündungen, Sepsis, Influenza, Covid-19, ARDS, oder eine Pneumonitis nach Immuntherapie bei Krebs geeignet ist.

Die Erfindung wird anhand der nachfolgenden Beispiele näher erläutert, die jedoch die Erfindung nicht einschränken.

### Beispiele

### Beispiel 1: Bestimmung von LysoPC in Plasma, Serum oder Blut.

LysoPC aus Plasma, Serum oder Blut wurde mit einer neu entwickelten Methode in einem Schritt extrahiert und anschließend mit Hilfe der HPTLC (high performance thin layer chromatography) bestimmt.

Die Extraktion von je 50 µl Probe erfolgte mit Hilfe der Salz-assistierten Extraktion. Hierzu wurde in einem 2 ml Twist-Top-Vial eine Keramikkugel (3,4 mm, Keramik) vorgelegt, und dann 50 µl dest. Wasser, 50 µl Probe, 192 µl konz. Ammoniumacetat-Lösung sowie 300 µl Acetonitril/1-Pentanol (2,5:1 v/v) zugegeben. Das Vial wird nach dem Verschließen mit Hilfe der dualen Zentrifugation (ZentriMix 380 R, Fa. Hettich, Tuttlingen) für 4 min bei 2000 rpm und 20 °C intensiv zentrifugal geschüttelt. Anschließend werden die Vials für 10 min bei 16.000 rpm zentrifugiert. Von der oberen Phase werden 200 µl abgenommen, in ein Rollrandgläschen mit Mikroeinsatz überführt, verbördelt und der HPTLC zugeführt.

Für die Bestimmung der LysoPC-Konzentrationen mit Hilfe der HPTLC werden fünf LysoPC- Standards (16, 40, 80, 120 und 160 µM LysoPC in Acetonitril/1-Pentanol (2,5:1 v/v.) sowie bis zu 12 Proben-Extrakte auf eine 20 x 10 cm Kieselgelplatte mithilfe des automatischen Auftragegeräts ATS4 (CAMAG, Schweiz) aufgetragen. Das aufgetragene Volumen ist jeweils 6 µl.

Nach dem Auftragen wird die Platte nach fünfminütiger Wartezeit, um ein gleichmäßiges Abdampfen des Lösungsmittels zu gewährleisten, 20 min in eine vorkonditionierten DC Kammer, die mit 35 ml Chloroform/ Methanol/ Wasser (65:35:4 v/v) und 200 µl 25% Ammoniaklösung befüllt ist, gestellt. In dieser Zeit wird eine Laufstrecke von ca. 9 cm erreicht. Zum Abdampfen des Laufmittels wird die Platte anschließend auf einer Thermoplatte bei 150 °C getrocknet und drei Mal für zwei Sekunden in eine Kupfersulfatlösung eingetaucht (83,3 g Kupfersulfat-Pentahydrat und 66,6 ml Phosphorsäure (85%) ad 1000 ml Wasser). Anschließend wird die Rückseite der Platte trocken getupft und diese für 10 min im vorgeheizten Trockenschrank bei 160 °C entwickelt. Nach erneutem Abkühlen auf Raumtemperatur wird die optische Dichte der LysoPC-Banden (Rf ca. 0,1) mit Hilfe der Auswerteeinheit (TLC Visualizer 2, CAMAG, Schweiz) bestimmt und die LysoPC-Konzentrationen durch Vergleich mit den Eichsubstanzen mit Hilfe der HPTLC Software "visionCATS" (Camag, Schweiz) ausgewertet. Wird der LysoPC-Wert nicht direkt aus Plasma, sondern aus Vollblut bestimmt, muss der Wert um den jeweiligen Hämatokrit-Wert sowie um den Faktor 1.3 korrigiert werden. Die inter-assay-Präzision der Bestimmung kann mit einem COV von 11,3 % als gut beschrieben werden.

### Beispiel 2: Bestimmung der Gesamtmenge des LysoPCs im Körper

Einem gesunden Probanden (männlich, 57 Jahre, 84 kg) mit einem Plasmavolumen von ca. 3,5 I wurden im nüchternen Zustand insgesamt 5 gekochte Eier á 60 g verabreicht und anschließend im Abstand von 15 oder 30 min über 3 h je 50 µl Blut aus der Fingerbeere entnommen. Die Bestimmung der Blut-LysoPC-Konzentration erfolgte wie in Beispiel 1 beschrieben.

Die Bestimmung der Plasma-LysoPC-Konzentration im nüchternen Zustand ergab 190 µM. Nach Aufnahme von 5 Eiern mit insgesamt 86,5 g Eigelb, welches ca. 5,8 g Phosphatidylcholin sowie 360 mg LysoPC enthält (Blesso, CH., Nutrients 7: 2731-2747 (2015)) steigt der LysoPC-Wert im Plasma rasch an und erreichte nach 75 min den höchsten Wert mit einer Steigerung des LysoPC-Spiegels um 70 %, um danach bis zum Ende der Messung auf einem konstanten Niveau von ca. 60-70 % über dem Ausgangs-LysoPC-Spiegel zu verbleiben.

Da sich zu Beginn der Messungen mit einem Startwert von 190 µM ca. 330 mg LysoPC in den 3,5 I Plasma befunden haben, entspricht ein Anstieg des Spiegels um 70 % einer zusätzlichen LysoPC-Menge von ca. 230 mg im Plasma.

Unter der Annahme, dass das gesamte durch die Eier zugeführte Phosphatidylcholin im GI-Trakt in LysoPC gespalten wird, ergibt sich zusammen mit dem ohnehin im Ei befindlichen LysoPC eine maximale LysoPC-Aufnahme von 4.300 mg.

Von der zugeführten LysoPC-Menge von 4.300 mg wurden folglich nur 230 mg im Plasma wiedergefunden (5,3 %). In erster grober Näherung kann daraus geschlossen werden, dass der Großteil (ca. 95 %) des zugeführten LysoPCs in tieferen Kompartimenten befindet und das das Plasma-Kompartiment und die tieferen Kompartimente (Organe, Gewebe) in einem schnellen Gleichgewicht stehen.

### Beispiel 3: Bestimmung der Gesamtmenge des LysoPCs im Körper

Einem gesunden Probanden (männlich, 57 Jahre, 84 kg) mit einem Plasmavolumen von ca. 3,5 I wurden im nüchternen Zustand 25 g reine Triglyceride (Margarine) verabreicht und anschließend im Abstand von 15 oder 30 min über 3 h je 50 µl Blut aus der Fingerbeere entnommen. Die Bestimmung der Blut-LysoPC-Konzentration erfolgte wie in Beispiel 1 beschrieben.

Die Bestimmung der Plasma-LysoPC-Konzentration im nüchternen Zustand ergab erneut 190 µM. Nach Aufnahme der Triglyceride fiel der LysoPC-Wert fast linear ab und erreichte den stärksten Abfall nach ca. 2 h (ca. -20 %) um danach bis zum Ende der Messung auf einem konstanten Niveau von ca. 20 % unter dem Ausgangs-LysoPC-Spiegel zu verbleiben.

Dieses Experiment wurde durchgeführt, da das Ergebnis in Beispiel 2 auch dadurch erklärt werden könnte, dass nur ein sehr geringer Anteil des aus dem PC im GI-Trakt gebildeten LysoPCs letztlich in tiefere Kompartimente übertragen wird und nur ein geringer Anteil ins Blut gelangt, der Großteil würde von den Enterozyten abgebaut. Um zu zeigen, dass dies nicht der Fall ist, wurde reines Triglycerid zugeführt, welches letztlich als Chylomikron via Lymphsystem ins Blut gelangt. Zum Aufbau von Chylomikronen bedarf es neben den Apolipoproteinen und den Triglyceriden ca. 10 % Phospholipide, die von den Enterozyten synthetisiert werden müssen. Hierzu bietet sich als sehr schneller Syntheseweg die Reacylierung von LysoPC aus dem Plasma an.

Da sich zu Beginn der Messungen mit einem Startwert von 190 µM ca. 330 mg LysoPC in den 3,5 I Plasma befunden haben, entspricht das Abfallen des Spiegels um 20 % einem Verlust von ca. 66 mg LysoPC im Plasma.

Unter der Annahme, dass das gesamte zugeführte Triglycerid in den Enterozyten in Chylomikronen überführt wurde und dass Chylomikronen bis zu 10 % aus Phospholipiden, meist Phosphatidylcholin bestehen (Stein, Y., Lipid-Review 5:38 (1988)), ergibt sich ein PC-Bedarf für die Herstellung der Chylomikronen von bis zu 2,5 g. Wird diese Menge von PC über die rasche Reacylierung von LysoPC synthetisiert, dann entspricht dies einer LysoPC-Entnahme aus dem System von 1.700 mg.

Von den der verbrauchten LysoPC-Menge von 1.700 mg wurden ca. 66 mg dem Plasma entnommen (3,9 %). In einer erste groben Näherung kann daraus geschlossen werden, dass der Großteil (ca. 96 %) des durch den Prozess der Chylomikronen-Synthese verbrauchten LysoPCs aus tieferen Kompartimenten entnommen wurde und dass sich ca. 96 % des Gesamt-Körper-LysoPC in diesen tieferen Kompartimenten aufhält und dass das Plasma-Kompartiment und die tieferen Kompartimente (Organe, Gewebe) in einem schnellen Gleichgewicht stehen.

Durch die Ergebnisse der Beispiele 2 und 3 konnte die LysoPC-Menge in tieferen Kompartimenten mit 95-96 % des Gesamt-Körper-LysoPCs abgeschätzt werden, d. h. 4-5 % befinden sich im Blutplasma.

Umgerechnet auf einen mittleren LysoPC-Wert von 300 µM bei Gesunden (s.o.) und einem Plasmavolumen von ca. 3,5 I befindet sich im Plasma ca. 500 mg LysoPC. Basierend darauf lässt sich die Menge des Gesamt-Körper-LysoPCs mit ca. 10 g grob abschätzen.

Basierend auf einer geschätzten Gesamt-Körper-Halbwertszeit für LysoPC von 2,5 h (s.o.) ergibt sich ein Tagesumsatz von ca. 50 g bei Gesunden, in pathologischen Situationen wie bei einer Krebserkrankung oder einer Entzündung dürfte der Wert deutlich höher liegen (s.o.).

### Beispiel 4: Bestimmung der LvsoPC-Konzentration vor und nach körperlicher Aktivität

Bei einer Gruppe von 8 Probanden (männlich) wurden die Plasma-LysoPC-Spiegel direkt vor und nach einem Marathonlauf untersucht. Die Werte vor dem Lauf lagen im Mittel bei 294,9 µM (222,3 - 367,9 µM). Durch den Lauf fielen die Spiegel im Mittel auf 213,6 µM, d. h. um 27,6 % (156,3 - 258,4 µM), die stärkste Abnahme betrug - 150 µM (-45 %). Der hier gemessene LysoPC-Abfall kann durch einen sehr hohen Verbrauch durch die langanhaltende körperliche Leistung erklärt werden und dass der für die sportliche Leistung notwendige Mehrverbrauch an LysoPC nicht mehr durch die normalen Mechanismen der LysoPC-Biosynthese kompensiert werden kann.

### Beispiel 5: Bestimmung des LysoPC-Anstiegs durch orale Aufnahme von alpha-Glvcerophosphocholin

Da bei der Freisetzung einer Fettsäure aus LysoPC im Gewebe gleichzeitig das wasserlösliche alpha-Glycerophosphocholin freigesetzt wird, bleibt dieses in wässerigen Körperkompartiment zurück. Um zu prüfen, ob alpha-GPC auch als Akzeptor für die Aufnahme von Fettsäuren, z. B. in der Leber, zur Verfügung stehen könnte, wurde die Veränderung des LysoPC-Spiegels nach Aufnahme von 600 mg alpha-GPC bestimmt.

Einem gesunden Probanden (männlich, 57 Jahre, 84 kg) mit einem Plasmavolumen von ca. 3,5 I wurden im nüchternen Zustand 600 mg alpha-GPC verabreicht und anschließend im Abstand von 15 oder 30 min über 2 h je 50 µl Blut aus der Fingerbeere entnommen. Die Bestimmung der Blut-LysoPC-Konzentration erfolgte wie in Beispiel 1 beschrieben.

Die Bestimmung der Plasma-LysoPC-Konzentration im nüchternen Zustand ergab erneut ca. 190 µM. Der LysoPC-Spiegel veränderte sich in der ersten Stunde nach der Aufnahme von alpha-GPC nicht, stieg dann aber in den nächsten 30 min um ca. 20 % an, um bis zum Ende der Messung konstant bei diesem Wert zu verweilen.

Da sich zu Beginn der Messungen mit einem Startwert von 190 µM ca. 330 mg LysoPC in den 3,5 I Plasma befunden haben, entspricht ein Anstieg des Spiegels um 20 % einer zusätzlichen LysoPC-Menge von ca. 66 mg im Plasma.

Unter der Annahme, dass sich nur ca. 5 % des Gesamt-Körper LysoPCs im Plasma befinden, bedeutet die Zunahme im Plasma von ca. 66 mg eine Gesamtzunahme von ca. 1.300 mg LysoPC bzw. 2,6 mmol LysoPC (MR= ca. 500 g/mol). Dies entspricht recht gut der molaren Menge, nämlich 2,3 mmol, des oral aufgenommenen alpha-GPCs (MR= 257,2 g/mol), was einen ersten Hinweis darauf gibt, das zusätzlich aufgenommenes alpha-GPC tatsächlich zu großen Anteilen wieder in LysoPC überführt wird. Dies zeigt, dass alpha-GPC bzw. LysoPC ein wichtiger systemischer Fettsäure-Carrier sein könnte.

### Beispiel 6: Bestimmung des LysoPC-Anstiegs durch orale Aufnahme von Glucose

Da die Aufnahme von Glucose u.a. auch zu einer Steigerung der Fettsäurebiosynthese in der Leber führt, wurde in diesem Experiment geprüft, ob diese zusätzliche Fettsäurebiosynthese mit einer Steigerung der LysoPC-Menge im Körper in Verbindung steht.

Einem gesunden Probanden (männlich, 57 Jahre, 84 kg) mit einem Plasmavolumen von ca. 3,5 I wurden im nüchternen Zustand 50 g Glucose verabreicht und anschließend im Abstand von je 15 oder 30 min je 50 µl Blut aus der Fingerbeere entnommen. Die Bestimmung der Blut-LysoPC-Konzentration erfolgte wie in Beispiel 1 beschrieben.

Die Bestimmung der Plasma-LysoPC-Konzentration im nüchternen Zustand ergab erneut ca. 190 µM. Der LysoPC-Spiegel stieg über 2 h um ca. 35 % an, spätere Messpunkte standen nicht zur Verfügung.

Da sich zu Beginn der Messungen mit einem Startwert von 190 µM ca. 330 mg LysoPC in den 3,5 I Plasma befunden haben, entspricht ein Anstieg des Spiegels um 35 % einer zusätzlichen LysoPC-Menge von ca. 116 mg im Plasma.

Unter der Annahme, dass sich nur ca. 5 % des Gesamt-Körper LysoPCs im Plasma befinden, bedeutet die Zunahme im Plasma von ca. 116 mg eine Gesamtzunahme von ca. 2.300 mg LysoPC bzw. 4,6 mmol LysoPC (MR= ca. 500 g/mol).

Dies zeigt, dass es auch möglich sein kann, dass durch die Bereitstellung von Fettsäuren in der Leber eine Steigerung der LysoPC-Biosynthese induziert wird. Insbesondere deutet dieser Befund erneut darauf hin, dass LysoPC ein wichtiger Fettsäure-Carrier sein könnte.

### Beispiel 7: Vergleich der LvsoPC-Werte über mehrere Tage

Die in den Beispielen 2, 3, 4 und 6 bestimmten LysoPC-Werte wurden über einen Zeitraum von 1 Woche mit immer dem gleichen gesunden Probanden (männlich, 57 Jahre, 84 kg) mit einem Plasmavolumen von ca. 3,5 I erhoben. Die Blutproben für die Startwerte wurden alle morgens gegen 9:00 Uhr im nüchternen Zustand (Nahrungskarrenz beginnend am Vorabend um ca. 19:00 Uhr) abgenommen.

Die befundenen Startwerte lagen überraschenderweise immer bei 190 µM LysoPC, Schwankungen wurden nicht beobachtet. Dies zeigt, dass die individuellen LysoPC-Werte zumindest bei einem gleichmäßigen Lebensstil zwar individuell unterschiedlich sein können, aber für einen bestimmten Menschen wiederum nur sehr wenig schwanken.

## Patentansprüche

**1.** Phospholipid und/oder Alpha-Glycerophosphocholin(alpha-GPC)-haltige Zusammensetzung zur Verwendung in der Behandlung von viralen und bakteriellen Lungenentzündungen und Sepsis.

**2.** Phospholipid- und/oder alpha-GPC haltige Zusammensetzung zur Verwendung in der Behandlung von viralen und bakteriellen Lungenentzündungen und Sepsis nach Anspruch 1, wobei die Zusammensetzung
(i) ein oder mehrere Acylglycerophospholipide, insbesondere Acylglycerophosphatidylcholine sowie deren Metaboliten wie Lyso-Phosphatidylcholine und alpha-Glycerophosphocholin enthält; und/oder
(ii) zur Behandlung, zur Vorbeugung, zur Unterstützung der Behandlung und zur Nachbehandlung von viralen und bakteriellen Lungenentzündungen und Sepsis, einschließlich Influenza, Covid-19, ARDS, Krebs und Pneumonitis nach Immuntherapie bei Krebs verwendet wird.

**3.** Phospholipid- und/oder alpha-GPC-haltige Zusammensetzung zur Verwendung in der Behandlung von viralen und bakteriellen Lungenentzündungen und Sepsis nach Anspruch 1 oder 2, wobei die Zusammensetzung eine oder mehrere der folgenden Verbindungen enthält:
(i) Acylglycerophospholipide ausgewählt aus 1,2-Diacylglycerophospholipiden, 1-Acylglycerophospholipiden und 2-Acylglycerophospholipiden mit gesättigten oder ungesättigten Acylresten, einschließlich Phosphatidylcholinen, Lyso-Phosphatidylcholinen und Lecithin, wobei diese Verbindungen synthetisch hergestellt oder aus natürlichen Quellen stammen und bevorzugt (a) Lecithin, besonders bevorzugt hydriertes Lecithin, und/oder (b) ein Phosphatidylcholin, besonders bevorzugt Dipalmitoylphosphatidylcholin sind; und/oder
(ii) Acylglycerophospholipide mit einer Struktur der Formel (I) 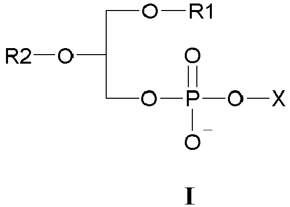 worin
R¹ und R² unabhängig voneinander ausgewählt sind aus H, Alkylcarbonyl-, Alkenylcarbonyl-, Alkinylcarbonyl-, Arylalkylcarbonyl- und Cycloalkylcarbonylresten, worin die Alkylreste geradkettig, verzweigt oder zyklisch, gesättigt oder ungesättigt und mit 1 bis 3 Resten R³ substituiert sein können und in den Alkylresten eines oder mehrere der C-Atome durch O oder NR⁴ ersetzt sein können,
X ausgewählt ist aus H, -(CH₂)ₙ-N(R⁴)₃⁺, -(CH₂)ₙ-CH(N(R⁴)₃⁺)-COO⁻ und -(CH₂)ₙ-CH(OH)-CH₂OH, wobei n eine ganze Zahl von 1 bis 5 ist, R³ unabhängig vom Auftreten weiterer R³-Reste ausgewählt ist aus H, Niederalkyl (worin die Niederalkylreste geradkettig, verzweigt oder zyklisch, gesättigt oder ungesättigt sein können), F, Cl, CN und OH, und R⁴ unabhängig vom Auftreten weiterer R⁴-Reste ausgewählt ist aus H, CH₃ und CH₂CH₃,
oder deren pharmazeutisch geeignete Salze sind; und/oder
(iii) Phosphatidylcholine der Formel (II) 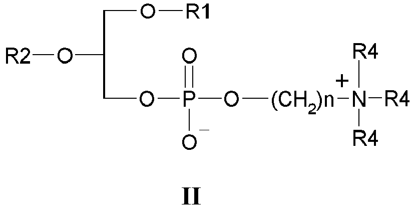 worin
R¹ und R² unabhängig voneinander H oder unverzweigte und unsubstituierte Alkylcarbonylreste sind, welche entweder gesättigt und dann bevorzugt ausgewählt sind aus Lauryl-, Myristyl-, Palmitoyl-, Stearyl-, Arachinyl-, Behenyl- und Lignocerylresten und ganz besonders bevorzugt aus Myristyl-, Palmitoyl-, Stearyl- und Arachniylresten, oder ungesättigt und dann bevorzugt ausgewählt sind aus Oleyl-, α-Linolenyl-, Eicosapentaenyl- und Docosahexaenyl-Resten,
R⁴ unabhängig voneinander CH₃ oder H sind, und
n 2 oder 3 ist; und/oder
(iv) Lyso-Phospholipide, Verbindungen der vorstehend gezeigten Verbindungen der Formeln (I) und (II), in denen eines von R¹ und R² H ist; und/oder
(v) Alpha-Glycerophosphocholin (alpha-GPC), eine Verbindung der Formel (II), in der R¹ und R² beide H sind.

**5.** Phospholipid- und/oder alpha-GPC-haltige Zusammensetzung zur Verwendung in der Behandlung von viralen und bakteriellen Lungenentzündungen und Sepsis nach Anspruch 3, wobei in der Verbindung der Formel (I) die Alkylcarbonylreste 10 bis 24 C-Atome aufweisen, gesättigt sind oder eine oder mehrere Doppelbindungen enthalten, wobei die Zahl der C-Atome bevorzugt ein Vielfaches von 2 ist und die Doppelbindungen nicht konjugiert sind, und wobei die Alkylreste besonders bevorzugt Fettsäurereste sind, die Niederalkylreste 1-3 C-Atome aufweisen und bevorzugt gesättigt sind, und n eine ganze Zahl von 1 bis 3 ist.

**5.** Phospholipid- und/oder alpha-GPC-haltige Zusammensetzung zur Verwendung in der Behandlung von viralen und bakteriellen Lungenentzündungen und Sepsis nach Anspruch 3, wobei in der Verbindung der Formel (I) R¹ und R² unabhängig voneinander H oder unverzweigte und unsubstituierte Alkylcarbonylreste sind, welche entweder gesättigt sind und dann bevorzugt ausgewählt sind aus Lauryl-(n-Dodecanyl-), Myristyl- (n-Tetradecanyl-), Palmitoyl- (n-Hexadecanyl-), Stearyl- (n-Octadecanyl-), Arachinyl- (n-Eicosanyl-), Behenyl- (n-Docosanyl-) und Lignoceryl- (n-Tetracosanyl-)resten und ganz besonders bevorzugt aus Myristyl-, Palmitoyl-, Stearyl- und Arachniylresten, oder ungesättigt sind und dann bevorzugt ausgewählt sind aus Oleyl- (18:1), α-Linolenyl- (18:3), Eicosapentaenyl- (20:5) und Docosahexaenyl (22:6)-Resten, R³ H ist, und X-(CH₂)₂-N(CH₃)₃⁺, -(CH₂)₂-NH₃⁺, oder -CH₂-CH(NH₃⁺)-COO⁻ ist.

**6.** Acylglycerophospholipid- und/oder alpha-GPC-haltige Zusammensetzung zur Verwendung in der Behandlung von viralen und bakteriellen Lungenentzündungen und Sepsis nach Anspruch 3, wobei das Phosphatidylcholin der Formel (II) ein Phosphatidylcholin (PC) mit gesättigten Fettsäureresten, insbesondere Dipalmitoylphosphatidylcholin (DPPC) ist und besonders bevorzugt Ei- oder Soja-Lecithin oder hydriertes Ei- oder Soja-Lecithin ist.

**7.** Phospholipid- und/oder alpha-GPC-haltige Zusammensetzung zur Verwendung in der Behandlung von viralen und bakteriellen Lungenentzündungen und Sepsis nach Anspruch 3, wobei das Lyso-Phospholipid
(i) eine Fettsäure mit einer Länge von mindestens C16,
(ii) eine gesättigte Fettsäure, oder
(iii) eine ω-3 oder ω-9 Fettsäure, die bevorzugt mindestens eine Länge von C18, besonders bevorzugt von C20 hat und insbesondere Eicosapentaensäure (20:5) oder Docosahexaensäure (22:6) ist,
ist.

**8.** Phospholipid- und/oder alpha-GPC-haltige Zusammensetzung zur Verwendung in der Behandlung von viralen und bakteriellen Lungenentzündungen und Sepsis nach einem oder mehreren der Ansprüche 1 bis 7, wobei der Anteil des Phospholipids oder Acylglycerophospholipids an den Gesamtlipiden in der Zusammensetzung mindestens 10 Gew-%, bevorzugt mindestens 40 Gew.-%, ganz besonders bevorzugt 100 Gew.-% beträgt.

**9.** Phospholipid- und/oder alpha-GPC-haltige Zusammensetzung zur Verwendung in der Behandlung von viralen und bakteriellen Lungenentzündungen und Sepsis nach einem oder mehreren der Ansprüche 1 bis 8, wobei
(i) das Acylglycerophospholipid der einzige vorhandene pharmakologisch aktive Wirkstoff der Zusammensetzung ist, besonders bevorzugt das einzige vorhandene GPL ist, wobei insbesondere nur Acylgylcerophosphocholin vorhanden ist; oder
(ii) LysoPC das einzige in der Zusammensetzung enthaltene Phospholipid ist; oder
(iii) alpha-GPC der einzige in der Zusammensetzung enthaltene Wirkstoff ist; oder
(iv) GPL, LysoPC und alpha-GPC als Kombinationen in der Zusammensetzung vorhanden sind, bevorzugt (a) AGPL und alpha-GPC (oral), (b) LysoPC und alpha-GPC (systemisch).

**10.** Phospholipid- und/oder alpha-GPC-haltige Zusammensetzung zur Verwendung in der Behandlung von viralen und bakteriellen Lungenentzündungen und Sepsis nach einem oder mehreren der Ansprüche 1 bis 8, wobei die Zusammensetzung weiterhin
(i) Triglyceride, Fettsäuremonoester, Wachse und/oder freie Fettsäuren enthält, wobei diese Lipide vorzugsweise antientzündliche und/oder nicht entzündliche Fettsäuren enthalten, besonders bevorzugt Öle, die wenig oder keine ω-6-Fettsäuren, aber dafür ω-3- oder ω-9-Fettsäuren oder gesättigte Fettsäuren enthalten; oder
(ii) alpha-GPC (oral oder iv); oder
(iii) alpha-GPC (oral oder iv) in Kombination mit den PL (siehe oben) oder mit Fischölen, oder anderen Ölen mit geeigneten Fettsäuren,
enthalten.

**11.** Phospholipid- und/oder alpha-GPC-haltige Zusammenfassung zur Verwendung in der Behandlung von viralen und bakteriellen Lungenentzündungen und Sepsis nach Anspruch 10, wobei in den Zusammensetzungen weiterhin einer oder mehrere der folgenden Wirkstoffe enthält: Albumin (bei systemischer Gabe); Glucose; Insulin; antientzündliche Wirkstoffen, antivirale Wirkstoffe; Proteine/AS, um den Muskelabbau zu reduzieren, wie z. B. Molkeprotein; und Carnitin.

**12.** Phospholipid- und/oder alpha-GPC-haltige Zusammensetzung zur Verwendung in der Behandlung von viralen und bakteriellen Lungenentzündungen und Sepsis nach einem oder mehreren der Ansprüche 1 bis 11, wobei die Zusammensetzung
(i) zur oralen oder intravenösen Gabe geeignet ist; und/oder
(ii) zur Gabe von Dosen von 2-300 mg AGPL/kg Körpergewicht bzw. mindestens 500 mg alpha-GPC pro Tag, vorzugsweise von wenigstens 20 mg AGPL/kg Körpergewicht pro Tag geeignet ist.

**13.** Phospholipid- und/oder alpha-GPC-haltige Zusammensetzung zur Verwendung in der Behandlung von viralen und bakteriellen Lungenentzündungen und Sepsis nach einem oder mehreren der Ansprüche 1 bis 12, wobei die Zusammensetzung als Emulsion, als Tabletten, Kapseln oder als Pulver zum Einrühren in Nahrungsmittel oder zur Direkteinnahme vorliegt.

**14.** Verfahren zur Behandlung von viralen und bakteriellen Lungenentzündungen und Sepsis, umfassend das Verabreichen einer Phospholipid- und/oder alpha-GPC-haltigen Zusammensetzung an einen Patienten mit Bedarf an einer solchen Behandlung.
